# EUROPÄISCHE PATENTSCHRIFT

(11) **EP 3 811 881 B1**
(45) Veröffentlichungstag und Bekanntmachung des Hinweises auf die Patenterteilung: **10.01.2024**
(21) Anmeldenummer: 19204495.6
(22) Anmeldetag: 22.10.2019
(51) Int. Cl.: A61B 17/88, A61B 17/00

(54) **VORRICHTUNG ZUM AUSTRAGEN VON KNOCHENZEMENT**
DEVICE FOR APPLYING BONE CEMENT
DISPOSITIF DE DISTRIBUTION DU CIMENT OSSEUX

(43) Veröffentlichungstag der Anmeldung: 28.04.2021
(73) Patentinhaber: Heraeus Medical GmbH, 61273 Wehrheim (DE)
(72) Erfinder: KLUGE, Thomas, 61273 Wehrheim (DE); VOGT, Sebastian, 61273 Wehrheim (DE)
(74) Vertreter: Heraeus IP

(56) Entgegenhaltungen:
- WO-A1-2019/200091
- US-A1- 2004 204 715
- US-A1- 2004 260 304
- US-A1- 2013 079 786

## Beschreibung

Die Erfindung betrifft eine Vorrichtung zum Austragen eines Knochenzements aus einem Behältnis, umfassend ein Verbindungselement, um die Vorrichtung mit dem Behältnis reversibel zu verbinden, ein stangenartiges Austragselement, welches zumindest partiell ein Außengewinde aufweist, ein Schaltsystem, welches zumindest partiell ein erstes Innengewinde aufweist, wobei das erste Innengewinde derart formschlüssig mit dem Außengewinde koppelbar ist, dass in einer ersten gekoppelten Schaltsystemposition das Austragselement axial in das Behältnis schraubbar ist und in einer zweiten entkoppelten Schaltsystemposition das Austragselement frei axial in das Behältnis schiebbar ist. Die Erfindung betrifft weiterhin ein Verfahren zum Austragen eines Knochenzements aus einem Behältnis mit einer Vorrichtung umfassend ein Verbindungselement, um die Vorrichtung mit dem Behältnis reversibel zu verbinden, ein stangenartiges Austragselement, welches zumindest partiell ein Außengewinde aufweist, ein Schaltsystem, welches zumindest partiell ein erstes Innengewinde aufweist, wobei das erste Innengewinde derart formschlüssig mit dem Außengewinde koppelbar ist, dass in einer ersten gekoppelten Schaltsystemposition das Austragselement axial in das Behältnis schraubbar ist und in einer zweiten entkoppelten Schaltsystemposition das Austragselement frei axial in das Behältnis schiebbar ist.

### Hintergrund der Erfindung

Es werden erhebliche Bemühungen unternommen, Vorrichtung und Verfahren zum Austragen von Knochenzement aufzuzeigen, mittels derer Knochenzement einfach, sicher und kostengünstig während orthopädischen Operationen, insbesondere während des Fixierens von künstlichen Hüft- und Kniegelenken mit Knochenmaterial, appliziert werden kann.

Üblicherweise wird der Knochenzement in Behältnissen bereitgestellt und mittels Austragspistolen aus den Behältnissen ausgetragen. Solche Austragspistolen sind beispielsweise in der US 5,638,997 B2 und US 4,338,925 A beschrieben. Die Austragspistolen verfügen über einen Abzugsmechanismus, wobei die Betätigung eines Abzugs ein Austragselement antreibt, welches den Knochenzement aus dem Behältnis austrägt. Nachteilig an solchen Austragspistolen ist die Schwierigkeit, genaue Mengen an Knochenzement aus dem Behältnis auszutragen, da die Menge des Knochenzements üblicherweise durch vollständige

Abzugshube oder zumindest in, durch die Mechanik der Austragspistole auferlegten, diskreten Volumeneinheiten abgegeben wird. Zudem fallen bei der Benutzung solcher Austragspistolen hohe Kosten an, welche sich zum einen durch die hohen Anschaffungskosten, als auch durch die anfallenden Desinfektionsschritte zur Wiederverwendung der teuren Applikationspistole, ergeben.

Es besteht im Markt der Wunsch zur Verbesserung und Kostensenkung von Vorrichtungen zum Austragen von Knochenzement.

In der US 6,796,987 B2 wird eine Vorrichtung zum Austragen von Knochenzement beschrieben, welche ein mit einem Außengewinde versehenes Austragselement aufweist, um durch eine Drehbewegung einen Knochenzement aus einem Behältnis auszutragen. Das Außengewinde koppelt dazu einseitig mit einem Ausschnitt eines Innengewindes eines Schaltelements, wobei das Innengewinde mittels einer Feder gegen das Außengewinde gedrückt wird. Durch einen konstanten Druck gegen das Schaltsystem kann das Innengewinde von dem Außengewinde entkoppelt werden, was eine axial freie Verschiebung des Austragselements innerhalb der Vorrichtung erlaubt.

Ein Nachteil der Vorrichtung ist, dass die axial freie Verschiebung ausschließlich bei Aufrechterhaltung eines konstanten Drucks gegen das Schaltsystem möglich ist. Dies erschwert dem Operateur die Bedienung der Vorrichtung, da eine Hand durch die Betätigung des Schaltsystems gebunden ist.

Ein weiterer Nachteil der Vorrichtung ist die einseitige Kopplung des Außengewindes mit dem Innengewinde. Dies führt zu einer einseitigen Materialbelastung und erhöht somit die Anfälligkeit von bruchbedingten Ausfällen der Vorrichtung.

Ein weiterer Nachteil der Vorrichtung ist eine schwache mechanische Kopplung zwischen Außengewinde und Innengewinde, welche ausschließlich durch die Spannkraft der Feder bestimmt wird. Insbesondere bei hochviskosen Knochenzementen reicht die Spannkraft der Feder nicht aus den Knochenzement auszutragen. Statt einem fortgeführten Einschrauben des Austragselements kommt es stattdessen zu einem Wegdrücken, und damit zu einem Entkoppeln, des Innengewindes von dem Außengewinde.

Hinzu kommt, dass die Vorrichtung viele bewegliche Bauteile umfasst, so dass die Gefahr von Fehlfunktionen, insbesondere hervorgerufen durch die Feder, erhöht ist.

### Aufgaben

Die Aufgabe der vorliegenden Erfindung ist es, einen oder mehrere der sich aus dem Stand der Technik ergebenden Nachteile zumindest teilweise zu überwinden.

Im Speziellen basiert die Erfindung auf dem Ziel, Vorrichtungen zum Austragen eines Knochenzements aus einem Behältnis bereitzustellen, welche einfach in der Handhabung und kostengünstig sind, sowie eine hohe mechanische Stabilität aufweisen. Die Vorrichtung soll sowohl für Behältnisse geeignet sein, in den ein Knochenzement vor dem Austragen unter Einwirkung von Unterdruck an einem Ende des Behältnisses gesammelt wird, als auch für Behältnisse, in denen ein Knochenzement vor dem Austragen undefiniert über ein Innenvolumen des Behältnisses verteilt ist. Für Behältnisse, in denen ein Knochenzement vor dem Austragen, beispielsweise unter Einwirkung von Unterdruck, an einem Ende, insbesondere einem Austragsende, gesammelt wird, soll die Vorrichtung den Arbeitsaufwand für den Anwender so gering wie möglich halten und einen möglichst schnellen Beginn des Austragens ermöglichen.

Es ist auch die Aufgabe der Erfindung, ein Verfahren bereitzustellen, mit dem ein Knochenzement aus einem Behältnis ausgetragen werden kann, mittels derer mindestens ein Teil der bereits beschriebenen Aufgaben zumindest zum Teil gelöst wird.

### Allgemeines

In der vorliegenden Beschreibung beinhalten Bereichsangaben auch die als Grenzen genannten Werte. Eine Angabe der Art "im Bereich von X bis Y" in Bezug auf eine Größe A bedeutet folglich, dass A die Werte X, Y und Werte zwischen X und Y annehmen kann. Einseitig begrenzte Bereiche der Art "bis zu Y" für eine Größe A bedeuten entsprechend als Werte Y und kleiner als Y.

Beschriebene Merkmale können mit dem Begriff "im Wesentlichen" verknüpft sein. Der Begriff "im Wesentlichen" ist so zu verstehen, dass unter realen Bedingungen und Fertigungstechniken eine mathematisch exakte Auslegung von Begrifflichkeiten wie "Überlagerung", "senkrecht", "Durchmesser" oder "auf einer Achse liegend" nie exakt, sondern nur innerhalb gewisser fertigungstechnischer Fehlertoleranzen gegeben sein kann. Beispielsweise schließen "im Wesentlichen parallele Achsen" einen Winkel von 85 Grad bis 95 Grad zueinander ein und "im Wesentlichen gleiche Volumen" umfassen eine Abweichung von bis zu 5 Volumen-%. Eine "im Wesentlichen aus Kunststoff bestehende Vorrichtung" umfasst beispielsweise einen Kunststoffanteil von ≥95 bis ≤100 Gewichts-%.

### Ausführliche Beschreibung

Der Gegenstand der Erfindung betrifft eine Vorrichtung zum Austragen eines Knochenzements aus einem Behältnis,
umfassend
ein Verbindungselement, um die Vorrichtung mit dem Behältnis reversibel zu verbinden,
ein stangenartiges Austragselement, welches zumindest partiell ein Außengewinde aufweist, ein Schaltsystem, welches zumindest partiell ein erstes Innengewinde aufweist,
wobei das erste Innengewinde derart formschlüssig mit dem Außengewinde koppelbar ist, dass in einer ersten gekoppelten Schaltsystemposition das Austragselement axial in das Behältnis schraubbar ist und

in einer zweiten entkoppelten Schaltsystemposition das Austragselement frei axial in das Behältnis schiebbar ist,
dadurch gekennzeichnet, dass
das erste Innengewinde das Austragselement manschettenartig umfasst und das Schaltsystem mittels einer rotatorischen Bewegung um eine Längsachse des Austragselements reversibel zwischen der ersten Schaltsystemposition und der zweiten Schaltsystemposition verschiebbar ist.

Die Vorrichtung weist ein Verbindungselement auf, um die Vorrichtung reversibel mit dem Behältnis, insbesondere mit einem offenen axialen Ende des Behältnisses, zu verbinden. Das Verbindungselement verbindet die Vorrichtung und das Behältnis derart, dass die Vorrichtung und das Behältnis einer gemeinsamen Achse liegen. Das Verbindungselement kann auf unterschiedliche Weise ausgestaltet sein, um die Vorrichtung reversibel mit einem Behältnis zu verbinden. In einer Ausgestaltungsform ist das Verbindungselement als Innengewinde ausgestaltet, um mit einem entsprechenden Außengewinde des Behältnisses eine Verbindung herzustellen. In einer weiteren Ausgestaltungsform ist das Verbindungselement als ein oder mehrere Einrasthaken ausgestaltet, welche an entsprechenden Vertiefungen an dem Behältnis einrasten. In einer weiteren, bevorzugten, Ausgestaltungsform sind die Vorrichtung und das Behältnis über einen Bajonettverschluss miteinander verbunden.

Die Vorrichtung weist ein stangenartiges Austragselement auf, um den Knochenzement aus dem Behältnis auszutragen. Dazu wird das Austragselement axial in das mit dem Verbindungselement verbundene offene Ende des Behältnisses eingeführt, was bei fortgesetztem Einführen zu einem Austragen des Knochenzementes aus einem, dem offenen Ende des Behältnisses gegenüberliegendem Austragsende führt. Um in das Behältnis einführbar zu sein, weist das Austragselement eine Außendurchmesser auf, welcher im Wesentlichen gleich oder kleiner einem Innendurchmesser des Behältnisses entspricht, sowie eine Länge, die ausreicht, um den Knochenzement vollständig aus dem Behältnis auszutragen.

Das Austragen des Knochenzements aus dem Behältnis durch das Einführen des Austragselement kann auf unterschiedliche Weise realisiert werden. In einer Ausgestaltungsform wirkt das Austragselement durch direkten physischen Kontakt auf den Knochenzement ein. Hierzu weist das Austragselement ein der Austragsseite des Behältnisses zugewandtes axiales Ende auf, welches einen Durchmesser aufweist, welcher im Wesentlichen dem Innendurchmesser des Behältnisses entspricht. Das derartige axiale Ende des Austragselements wirkt bei einem Verschieben in das Behältnis derart mit einer Innenwand des Behältnisses zusammen, dass der Knochenzement aus dem Behältnis ausgepresst wird. In einer weitere, bevorzugten, Ausgestaltungsform wirkt das Austragselement auf einen im Behältnis befindlichen Austragskolben, welcher sich räumlich zwischen dem Austragselement und dem Knochenzement befindet, ein. Der Austragskolben weist einen Austragskolbendurmesser auf, welcher im Wesentlichen dem Innendurchmesser des Behältnisses entspricht. Ein dem Austragskolben zugewandtes Ende des Austragselements verschiebt den Austragskolben mit fortgeführtem Einbringen des Austragselements in das Behältnis in Richtung der Austragsseite des Behältnisses und presst somit den Knochenzement aus dem Behältnis aus.

Die Vorrichtung weist ein Schaltsystem auf. Das Schaltsystem dient der Kontrolle der Bewegungsart des Austragselements in das Behältnis. Dazu weist das Schaltsystem zwei Schaltsystempositionen auf, wobei eine erste Schaltsystemposition ausschließlich eine Drehbewegung und eine zweite Schaltsystemposition eine Schiebbewegung des Austragselements in das Behältnis ermöglicht.

Um die Bewegungsart des Austragselements durch die Einstellung in eine der zwei Schaltsystemposition zu kontrollieren, weist das Austragselement zumindest partiell ein Außengewinde und das Schaltsystem zumindest partiell ein erstes Innengewinde auf. In der ersten Schaltsystemposition sind das Außengewinde und das erste Innengewinde miteinander gekoppelt und wirken form- und/oder kraftschlüssig derart zusammen, dass eine relative Drehbewegung von Austragselement und Schaltsystem zueinander das Austragselement in das Behältnis einschraubt. Das Außengewinde und das erste Innengewinde wirken in der ersten Schaltsystemposition derart zusammen, dass ein Einbringen des Austragselements in das Behältnis ausschließlich über eine Drehbewegung erfolgen kann. Um die Drehbewegung für den Anwender der Vorrichtung zu erleichtern, kann das Austragselement ein Schraubmittel, beispielsweise einen länglichen, an die Innenfläche einer Hand angepassten Griff, aufweisen. In der zweiten Schaltsystemposition sind das Außengewinde und das erste Innengewinde entkoppelt, so dass das Austragselement durch eine Schiebebewegung frei axial in das Behältnis verbringbar ist. In der zweiten Schaltsystemposition ist keine Drehbewegung zum Einbringen des Austragselements in das Behältnis notwendig.

Das erste Innengewinde umfasst das Austragselement, und damit auch das Außengewinde, manschettenartig. Manschettenartig bedeutet, dass das erste Innengewinde sowohl in der ersten Schaltsystemposition, als auch in der zweiten Schaltsystemposition, den gesamten Querschnittsumfang des Austragselements, und nicht nur einen Teilbereich davon, umfasst. Ein Vorteil der manschettenartigen Ausgestaltung des Innengewindes ist eine hohe mechanische Stabilität der Vorrichtung, welche insbesondere beim Einschrauben des Austragselements in der ersten Schaltsystemposition unter Austragen eines hochviskosen Knochenzements erforderlich ist. Die feste manschettenartige Umfassung verhindert ein unbeabsichtigtes Entkoppeln von Außengewinde und erstem Innengewinde, wie es bei nur abschnittsweiser Kopplung von Außengewinde und Innengewinde möglich wäre. Ein weiterer Vorteil ist die gleichmäßige mechanische Belastung des Austragselements, welche sich über die sich über die gesamte Querschnittsfläche des Austragselements erstreckt und damit zur mechanischen Stabilität der Vorrichtung beiträgt.

Das Schaltsystem ist durch eine rotatorische Bewegung um eine Längsachse des Austragselements reversibel zwischen erster Schaltsystemposition und zweiter Schaltsystemposition verschiebbar. Das Schaltsystem verbleibt in der eingestellten Schaltsystemposition, so lange keine externe Krafteinwirkung, beispielsweise durch den Anwender der Vorrichtung, auf das Schaltsystem einwirkt. Die Vorrichtung verfügt über kein Rückstellelement, wie beispielsweise eine Feder, welche das Schaltsystem selbstständig und ohne externe Krafteinwirkung, wie beispielsweise durch den Anwender der Vorrichtung, von der einen Schaltsystemposition in die andere Schaltsystemposition verbringt. Ein Vorteil eines derartigen Schaltsystems ist, dass der Anwender nicht durchgängig Kraft gegen ein Rückstellelement, wie beispielsweise eine Feder, aufbringen muss und somit beide Hände zum Hantieren der Vorrichtung und des Behälters zur freien Verfügung hat. Dies erleichtert dem Anwender die Handhabung der Vorrichtung. Ein weiterer Vorteil ist, dass ein derartiges Schaltsystem durch die geringe Anzahl an benötigten, insbesondere beweglichen, wie beispielsweise einem Rückstellelement, Bauteilen eine hohe mechanische Stabilität aufweist und damit ein verringertes Risiko von Fehlfunktionen aufweist.

Eine Ausgestaltungsform der Vorrichtung ist dadurch gekennzeichnet, dass das Schaltsystem einen ersten Hohlzylinder aufweist, welcher das erste Innengewinde, und einen, mit dem ersten Hohlzylinder axial verbundenen, zweiten Hohlzylinder aufweist, wobei der erste Hohlzylinder und der zweite Hohlzylinder derart verbunden sind, dass das Schaltsystem mittels einer gegenläufigen rotatorischen Bewegung des ersten Hohlzylinder gegen den zweiten Hohlzylinder um die Längsachse reversibel zwischen der ersten Schaltsystemposition und der zweiten Schaltsystemposition verschiebbar ist.

Das Schaltsystem weist einen ersten Hohlzylinder und einen zweiten Hohlzylinder auf. Unter einem Hohlzylinder ist ein rohrartiges Element zu verstehen, welches einen Innenraum und eine den Innenraum umgebende Elementwand umfasst. Der Hohlzylinder besitzt senkrecht zu einer Längsachse einen Querschnitt, wobei der Querschnitt unterschiedliche Formen annehmen kann. Beispielsweise kann der Querschnitt oval, quadratisch, fünfeckig, sechseckig, unregelmäßig oder kreisförmig sein. Ein kreisförmiger Querschnitt ist aufgrund der guten Handhabbarkeit für den Anwender bevorzugt, wobei der erste Hohlzylinder und/oder der zweite Hohlzylinder unterschiedlich große Querschnitte sowohl im Vergleich gegeneinander, als auch innerhalb eines Hohlzylinder aufweisen können. In einer Ausgestaltungsform ist es bevorzugt, dass der erste Hohlzylinder und/oder der zweite Hohlzylinder auf einer Außenseite Strukturen aufweisen, die die gegenläufige Drehung des ersten Hohlzylinders gegen den zweiten Hohlzylinder für den Anwender erleichtern. In einer Ausgestaltungsform weisen der erste Hohlzylinder und/oder der zweite Hohlzylinder auf der jeweiligen Außenseite Noppen auf. In einer weiteren, bevorzugten, Ausgestaltungsform, weisen der erste Hohlzylinder und/oder der zweite Hohlzylinder mindestens eine entlang der Längsachse der Vorrichtung verlaufende Wulst auf. Wülste sind bevorzugt, da dadurch die Griffigkeit der Außenfläche der Hohlzylinder, insbesondere beim Tragen von Handschuhen unter Operationsbedingungen, erhöht wird. Dies erleichtert zum einen das Verbringen des Schaltsystem in die erste Schaltsystemposition und die zweite Schaltsystemposition und senkt das Risiko des Anwenders mit einer oder beiden Händen während der rotatorischen Bewegung abzurutschen.

Der erste Hohlzylinder weist das Innengewinde auf und ist axial derart mit dem zweiten Hohlzylinder verbunden, dass eine gegenläufige Drehung der beiden Hohlzylinder um die Längsachse möglich ist. Der erste Hohlzylinder und der zweite Hohlzylinder können auf unterschiedliche Weise miteinander verbunden sein um eine gegenläufige Drehung zu ermöglichen. Beispiele für mögliche Verbindungsarten sind Gewinde, Steckverbindungen oder Bajonettverschlüsse.

Die gegenläufige Drehbarkeit des ersten Hohlzylinders gegen den zweiten Hohlzylinder kann auf unterschiedliche Wiese ausgestaltet sein. In einer Ausgestaltungsform können der erste Hohlzylinder und der zweite Hohlzylinder beliebig weit in eine Drehrichtung gegeneinander verdreht werden und bei dieser fortgeführten gegenläufigen Drehung das Schaltsystem abwechselnd in die erste Schaltsystemposition und die zweite Schaltsystemposition verbringen. In einer weiteren, bevorzugten, Ausgestaltungsform sind der erste Hohlzylinder und der zweite Hohlzylinder nicht beliebig weit gegeneinander in eine Drehrichtung drehbar, sondern die Drehbarkeit in eine Drehrichtung ist durch einen Anschlag begrenzt. In einer Ausgestaltungsform ist die Drehbarkeit durch genau einen Anschlag begrenzt, so dass das Schaltsystem durch eine gegenläufige Drehung des ersten Hohlzylinders und des zweiten Hohlzylinders in eine Drehrichtung bis zu diesem Anschlag in die erste Schaltsystemposition verbringbar ist und durch eine gegenläufige Drehung in die entgegengesetzte Drehrichtung bis zu diesem Anschlag in die zweite Schaltsystemposition verbringbar ist. In einer weiteren, bevorzugten, Ausgestaltungsform weist das Schaltsystem zwei Anschläge auf, so dass eine gegenläufige Drehbewegung des ersten Hohlzylinders und den zweiten Hohlzylinder bis zum ersten Anschlag das Schaltsystem in die erste Schaltsystemposition verbringt und eine entgegengesetzte Drehbewegung bis zum zweiten Anschlag das Schaltsystem in die zweite Schaltsystemposition verbringt. Durch das Vorhandensein mindestens eines Anschlags erhält der Anwender eine deutliche physische Rückmeldung, ob ein Umschalten der Schaltsystemposition stattgefunden hat. Vorzugsweise ist die jeweils eingestellte Schaltsystemposition auch durch optische Markierungen, beispielsweise durch Farbmarkierungen, Hervorhebungen oder Einkerbungen, am ersten Hohlzylinder und/oder am zweiten Hohlzylinder für den Anwender ablesbar.

Der erste Hohlzylinder und der zweite Hohlzylinder können auf unterschiedliche Weise zusammenwirken, um das Schaltsystem mittels einer gegenläufigen rotatorischen Bewegung des ersten Hohlzylinder gegen den zweiten Hohlzylinder um die Längsachse des Austragselements reversibel zwischen der ersten Schaltsystemposition und der zweiten Schaltsystemposition umzuschalten.

Eine Ausgestaltungsform der Vorrichtung ist dadurch gekennzeichnet, dass der zweite Hohlzylinder zumindest partiell ein zweites Innengewinde aufweist und das Außengewinde des Austragselements zumindest eine entlang der Längsachse verlaufende Außengewindenut, das erste Innengewinde zumindest eine entlang der Längsachse verlaufende erste Innengewindenut und das zweite Innengewinde zumindest eine entlang der Längsachse verlaufenden zweite Innengewindenut aufweist.

In dieser Ausgestaltungsform ist sowohl der erste Hohlzylinder als auch der zweite Hohlzylinder manschettenartig um das Austragselement angeordnet, so dass das erste Innengewinde und das zweite Innengewinde mit dem Außengewinde form- und/oder kraftschlüssig zusammenwirken können. In einer Ausgestaltungsform grenzt das zweite Innengewinde im Wesentlichen direkt axial an das erste Innengewinde, so dass das zweite Innengewinde eine Verlängerung des ersten Innengewindes darstellt. In einer weiteren Ausgestaltungsform besteht zwischen erstem Innengewinde und zweitem Innengewinde eine Lücke, wobei die Lücke eine kleinere axiale Erstreckung als das Außengewinde aufweist, so dass das Außengewinde zu jeder Zeit zumindest mit dem ersten Innengewinde oder dem zweiten Innengewinde form- und/oder kraftschlüssig zusammenwirken kann.

In der ersten Schaltsystemposition wirken das erste Innengewinde und/oder das zweite Innengewinde form- und/oder kraftschlüssig mit dem Außengewinde zusammen. Dadurch ist das Außengewinde immer zumindest mit einem der beiden Innengewinde gekoppelt und ein axiales Einbringen des Austragselements in das Behältnis ist ausschließlich durch eine Drehbewegung möglich.

Um in der zweiten Schaltsystemposition das Außengewinde vom ersten Innengewinde und zweiten Innengewinde zu entkoppeln, weisen das Außengewinde zumindest eine Außengewindenut, das erste Innengewinde zumindest eine erste Innengewindenut und das zweite Innengewinde zumindest eine zweite Innengewindenut auf. Die jeweiligen Gewindenuten verlaufen jeweils entlang der Längsachse des Austragselements. Die zumindest eine erste Innengewindenut und die zumindest eine zweite Innengewindenut erstrecken sich jeweils über die gesamte axiale Erstreckung des erste Innengewindes und des zweiten Innengewindes. Die zumindest eine Außengewindenut erstreckt sich zumindest so lang über das Außengewinde, wie das Austragselement in das Behältnis zum Vollständigen Austragen des Knochenzements eingebracht werden muss, wobei die Erstreckung ein dem Behältnis zugewandtes Ende des Austragselements umfasst. Entlang der jeweiligen Gewindenuten ist die Höhe der Gewindeflanken der jeweiligen Gewinde verringert. In einer Ausgestaltungsform ist die Höhe der Gewindeflanken auf Null reduziert, so dass das jeweilige Gewinde entlang der Gewindenut eine vollständige Unterbrechung aufweist. Eine Reduktion der Höhe der Gewindeflanke auf Null bedeutet, dass die Gewindenut sich auf einer Höhe mit einer tiefsten Einkerbung eines Gewindeganges des jeweiligen Gewindes befindet. In einer weiteren Ausgestaltungsform ist das jeweilige Gewinde entlang der Gewindenut nicht auf Null reduziert, sondern nur im Vergleich zu der Gewindeflanke außerhalb der Gewindenut, beispielsweise auf 50 % der Höhe, reduziert. In einer weiteren Ausgestaltungsform ist das jeweilige Gewinde entlang der Gewindenut über die tiefste Einkerbung des Gewindeganges hinaus reduziert.

Im jeweiligen Bereich der Gewindenuten findet keine form- und/oder kraftschlüssige Wechselwirkung zwischen dem Außengewinde und dem ersten Innengewinde und/oder dem zweiten Innengewinde statt. In der zweiten Schaltsystemposition sind die zumindest eine erste Innengewindenut und die zumindest eine zweite Innengewindenut derart zueinander angeordnet, dass das Austragselement frei axial in das Behältnis schiebbar ist.

Eine Ausgestaltungsform der Vorrichtung ist dadurch gekennzeichnet, dass die zumindest eine Außengewindenut das Außengewinde in Außengewindeabschnitte, die zumindest eine erste Innengewindenut das erste Innengewinde in erste Innengewindeabschnitte und die zumindest eine zweite Innengewindenut das zweite Innengewinde in zweite Innengewindeabschnitte entlang der Längsachse unterteilt.

Ein Gewindeabschnitt ist ein Bereich des jeweiligen Gewindes mit einer maximalen Höhe der jeweiligen Gewindeflanke, welcher nicht von einer Gewindenut durchzogen ist. Die Gewindeabschnitte sind derart ausgestaltet, dass ein form- und/oder kraftschlüssiges Zusammenwirken der Außengewindeabschnitte sowie der ersten Innengewindeabschnitte und/oder der zweiten Innengewindeabschnitte möglich ist. In der ersten Schaltsystemposition wirken die Außengewindeabschnitte mit den ersten Innengewindeabschnitten und/oder den zweiten Innengewindeabschnitten form- und/oder kraftschlüssig zusammen, so dass das Austragselement ausschließlich in das Behältnis durch eine Drehbewegung schraubbar ist. In der zweiten Schaltsystemposition ist die zumindest eine erste Innengewindenut und die zumindest eine zweite Innengewindenut derart zueinander angeordnet, dass die Außengewindeabschnitte entlang der ersten Innengewindenut und der zweiten Innengewindenut frei axial in der Vorrichtung verschiebbar sind.

Um ein freies axiale Verschieben des Austragselements in das Behältnis zu ermöglichen, ist eine Ausgestaltungsform der Vorrichtung dadurch gekennzeichnet, dass eine radiale Erstreckung der zumindest einen Außengewindenut, der zumindest einen ersten Innengewindenut und der zumindest einen zweiten Innengewindenut gleich oder größer einer radialen Erstreckung des Außengewindeabschnittes, des ersten Innengewindeabschnittes und des zweiten Innengewindeabschnittes ist.

Dadurch ist eine freie axiale Verschiebung der Außengewindeabschnitte entlang der axialen Erstreckung der ersten Innengewindenut und der zweiten Innengewindenut sowie gleichzeitig eine freie axiale Verschiebung der ersten Innengewindeabschnitte und der zweiten Innengewindeabschnitte entlang der axialen Erstreckung der Außengewindenut möglich.

Um ein form- und/oder kraftschlüssiges Zusammenwirken von Außengewinde, insbesondere von den Außengewindeabschnitten, mit dem ersten Innengewinde und/oder dem zweiten Innengewinde, insbesondere mit den ersten Innengewindeabschnitten und/oder den zweiten Innengewindeabschnitten, in der ersten Schaltsystemposition zu ermöglichen, ist eine Ausgestaltungsform der Vorrichtung dadurch gekennzeichnet, dass das Außengewinde, das erste Innengewinde und das zweite Innengewinde, insbesondere die Außengewindeabschnitte, die ersten Innengewindeabschnitte und die zweiten Innengewindeabschnitte, einen im Wesentlichen gleichen Steigungswinkel aufweisen.

Das Außengewinde sowie das erste Innengewinde und das zweite Innengewinde können eine unterschiedliche Anzahl von jeweiligen Gewindenuten, und damit einhergehend eine unterschiedliche Anzahl von jeweiligen Gewindeabschnitten, mit unterschiedlichen radialen Ausdehnungen aufweisen. Allerdings müssen, damit eine freie axiale Verschiebung des Austragselement in das Behältnis in der zweiten Schaltsystemposition möglich ist, die jeweiligen Gewinde räumlich derart angeordnet werden können, dass eine Verschiebung der Außengewindeabschnitte entlang der ersten Innengewindenut und der zweiten Innengewindenut und gleichzeitig eine Verschiebung der ersten Innengewindeabschnitte und der zweiten Innengewindeabschnitte entlang der Außengewindenuten möglich ist.

Eine Ausgestaltungsform der Vorrichtung ist dadurch gekennzeichnet, dass das Außengewinde, das erste Innengewinde und das zweite Innengewinde jeweils eine gleiche Anzahl von Außengewindenuten, ersten Innengewindenuten und zweiten Innengewindenuten mit der jeweils im Wesentlichen gleichen radialen Erstreckung aufweist.

Ein Vorteil dieser Ausgestaltungsform ist eine hohe mechanische Stabilität der Vorrichtung und eine gleichmäßige Kraftverteilung beim Austragen des Knochenzements auf die jeweiligen Gewinde.

In einer Ausgestaltungform weisen die jeweiligen Gewindeabschnitte die gleiche radiale Erstreckung wie die jeweiligen Gewindenuten auf. In einer weiteren, bevorzugten, Ausgestaltungform weisen die jeweiligen Gewindeabschnitte eine geringe, beispielsweise um bis zu 1 mm geringere, radiale Erstreckung als die jeweiligen Gewindenuten auf, wodurch dem Anwender die freie axiale Verschiebung des Austragselements in der zweiten Schaltsystemposition aufgrund einer vereinfachten räumlichen Positionierung des Austragelements innerhalb des Schaltsystems erleichtert wird.

Beispielsweise weisen die jeweiligen Gewinde zwei bis zwölf jeweilige Gewindenuten auf, wobei eine die Kraft umso gleichmäßiger auf die jeweiligen Gewinde verteilt wird, je höher die Anzahl der Gewindenuten ist. Die gleichmäßigere Kraftverteilung resultiert in einer erhöhten mechanischen Stabilität der Vorrichtung. Mit erhöhter Anzahl an Gewindenuten steigt auch die Schwierigkeit der passenden räumlichen Positionierung des Austrageelements innerhalb des Schaltsystems. Als guter Kompromiss zwischen hoher mechanischen Stabilität und Einfachheit der räumlichen Positionierung hat sich eine Gewindenutenanzahl von sechs bis zehn, insbesondere von acht Gewindenuten herausgestellt.

Eine Ausgestaltungsform der Vorrichtung ist dadurch gekennzeichnet, dass in der zweiten Schaltsystemposition die zumindest eine erste Innengewindenut und die zumindest eine zweite Innengewindenut auf einer im Wesentlichen gemeinsamen Achse liegen.

Dadurch ist ein Entkoppeln von Außengewinde und ersten Innengewinde sowie zweitem Innengewinde, und damit ein vollständiges reversibles Einschieben des Austragselements ohne Drehungen um die Längsachse des Austragelements in das Behältnis möglich, was dem Anwender die Bedienung der Vorrichtung erleichtert.

Um das Außengewinde von dem ersten Innengewinde und dem zweiten Innengewinde zu entkoppeln, und damit das Austragselement frei axial zu verschieben, ist eine Ausgestaltungsform der Vorrichtung dadurch gekennzeichnet, dass in der zweiten Schaltsystemposition die zumindest eine erste Innengewindenut und die zumindest eine zweite Innengewindenut derart zu dem Außengewinde ausgerichtet sind, insbesondere auf einer im Wesentlichen gemeinsamen Achse liegen, dass das Außengewinde frei axial in die erste Innengewindenut und die zweite Innengewindenut schiebbar ist, um das Austragselement frei axial in das Behältnis zu schieben.

Eine Ausgestaltungsform der Vorrichtung ist dadurch gekennzeichnet, dass in der ersten Schaltsystemposition die zumindest eine erste Innengewindenut und die zumindest eine zweiten Innengewindenut nicht auf einer gemeinsamen Achse liegen.

Dadurch ist das Außengewinde mit dem ersten Innengewinde und/oder dem zweiten Innengewinde form- und/oder kraftschlüssig verbunden und das Austragselement in der Folge ausschließlich durch Einschrauben in das Behältnis mittels Drehbewegung zu verbringen.

Um das Außengewinde mit dem Schaltsystem zu koppeln, ist eine Ausgestaltungform der Vorrichtung dadurch gekennzeichnet, dass in der ersten Schaltsystemposition die zumindest eine erste Innengewindenut und die zumindest eine zweite Innengewindenut nicht auf einer gemeinsamen Achse liegen, so dass das Außengewinde mit dem ersten Innengewinde und dem zweiten Innengewinde derart miteinander koppelt, dass das Austragselement axial in das Behältnis schraubbar ist.

Dies hat zur Folge, dass das erste Innengewinde, insbesondere der zumindest eine erste Innengewindeabschnitt, und/oder das zweite Innengewinde, insbesondere der zumindest eine zweite Innengewindeabschnitt, mit dem Außengewinde, insbesondere mit dem zumindest einem Außengewindeabschnitt form- und/oder kraftschlüssig zusammenwirken und somit miteinander gekoppelt sind.

Die Vorrichtung kann aus unterschiedlichen Materialien oder Materialkombinationen aufgebaut sein.

Eine Ausgestaltungsform der Vorrichtung ist dadurch gekennzeichnet, dass die Vorrichtung aus Kunststoff, insbesondere aus einem kompostierbaren Kunststoff, bevorzugt aus einem Glasfaser-verstärktem Kunststoff, Metall oder aus einer Kombination von Kunststoff, insbesondere aus einem kompostierbaren Kunststoff, bevorzugt aus einem Glasfaser-verstärktem Kunststoff und Metall gefertigt ist.

Kunststoffe sind aufgrund der geringen Kosten und einfachen Herstellweise bevorzugt. Die Verwendung von Kunststoffen ermöglicht eine ökonomisch tragbare Einmalnutzung der Vorrichtung, wodurch, zusätzlich zu den bereits geringen Anschaffungskosten, die kostenintensiven Desinfektionsschritte zur Wiederverwendung entfallen. Um die mechanische Stabilität der Kunststoffe zu erhöhen sind insbesondere mit Glasfasern verstärkte Kunststoffe bevorzugt. Aus ökologischen Gründen sind insbesondere kompostierbare Kunststoffe bevorzugt. Beispiele für Kunststoffe sind Polyamide, wie Polyamid 12 oder Polyamid 6, und Polyimide.

Um einzelnen, selektiven Abschnitten der Vorrichtung eine besonders hohe mechanische Belastbarkeit zu gewähren, können diese Abschnitte aus Metallen gefertigt sein. Beispiele für Metalle sind Eisen, Stahl, Kupfer oder Aluminium oder Legierungen aus diesen Metallen.

Das Verbindungselement kann an unterschiedlichen Stellen der Vorrichtung angebracht sein.

Aufgrund der einfachen Handhabung und der möglichst geringen Anzahl an Bauteilen ist eine Ausgestaltungsform der Vorrichtung dadurch gekennzeichnet, dass der erste Hohlzylinder das Verbindungselement umfasst.

Der Gegenstand der vorliegenden Erfindung betrifft auch ein Verfahren zum Austragen eines Knochenzements aus einem Behältnis mit einer Vorrichtung umfassend ein Verbindungselement, um die Vorrichtung mit dem Behältnis reversibel zu verbinden,
ein stangenartiges Austragselement, welches zumindest partiell ein Außengewinde aufweist, ein Schaltsystem, welches zumindest partiell ein erstes Innengewinde aufweist,
wobei das erste Innengewinde derart formschlüssig mit dem Außengewinde koppelbar ist, dass in einer ersten gekoppelten Schaltsystemposition das Austragselement axial in das Behältnis schraubbar ist und
in einer zweiten entkoppelten Schaltsystemposition das Austragselement frei axial in das Behältnis schiebbar ist,
zumindest umfassend die Schritte
   a) Verbinden der Vorrichtung mittels des Verbindungselements an dem Behältnis;
   b) Verbringen des Schaltsystems in die zweite Schaltsystemposition;
   c) axiales Verschieben des Austragselements in das Behältnis;
   d) Verbringes des Schaltsystems in die zweite Schaltsystemposition durch eine rotatorische Bewegung um eine Längsachse des Austragselements;
   e) Austragen des Knochenzements aus dem Behältnis durch ein Einschrauben des Austragselements in das Behältnis.

Eine weitere Ausgestaltung des Verfahrens ist dadurch gekennzeichnet, dass die rotatorische Bewegung in Schritt d) einen ersten Hohlzylinder des Schaltsystems gegen einen zweiten Hohlzylinder des Schaltsystems rotatorisch verschiebt. Ein Vorteil des beschriebenen Verfahrens ist, dass der Anwender die Vorrichtung, insbesondere das Schaltsystem, nicht durch dauerhafte Kraftaufwendung, beispielsweise gegen ein Rückstellelement, wie beispielsweise eine Feder, in der zweiten Schaltsystemposition halten muss. Somit stehen dem Anwender beide Hände zum Hantieren der Vorrichtung, insbesondere zum axialen Verschieben des Austragselements in das Behältnis, zur Verfügung, was die Durchführung des Verfahrens vereinfacht.

Eine weitere Ausgestaltung des Verfahrens ist dadurch gekennzeichnet, dass der erste Hohlzylinder das erste Innengewinde und der zweite Hohlzylinder ein zweites Innengewinde aufweist,
wobei das Außengewinde zumindest eine Außengewindenut,
das erste Innengewinde zumindest eine erste Innengewindenut und
das zweite Innengewinde zumindest eine zweite Innengewindenut umfassen, und die zumindest eine Außengewindenut das Außengewinde in Außengewindeabschnitte,
die zumindest eine erste Innengewindenut das erste Innengewinde in erste Innengewindeabschnitte und
die zumindest eine zweite Innengewindenut das zweite Innengewinde in zweite Innengewindeabschnitte jeweils entlang der Längsachse unterteilt und wobei die zumindest eine erste Innengewindenut und die zumindest eine zweite Innengewindenut in der zweiten Schaltsystemposition auf einer im Wesentlichen gemeinsamen Achse angeordnet sind, so dass in Schritt c) die Außengewindeabschnitte entlang der gemeinsamen Achse in das Behältnis verschoben werden.

Da die zumindest eine erste Innengewindenut und die zumindest eine zweite Innengewindenut in der zweiten Schaltsystemposition auf einer im Wesentlichen gemeinsamen Achse liegen, kann das axiale Verschieben des Austragselement in Schritt c) durch ein einfaches Eindrücken des Anwenders in das Behältnis durchgeführt werden. Dabei hat der Anwender beide Hände zur Verfügung, was die Durchführung des Verfahrens vereinfacht. Zudem muss der Anwender keinen zusätzlichen, durch die Vorrichtung verursachten zusätzlichen Kräfte, wie beispielsweise ein Drücken gegen ein Rückstellelement, aufwenden, um das Austragselement an die gewünschte Position zu verbringen. Ist das Austragselement an der gewünschten Position innerhalb des Behältnisses, verbringt der Anwender das Schaltsystem durch eine weitere rotatorische Bewegung des ersten Hohlzylinders gegen den zweiten Hohlzylinder in die erste Schaltsystemposition. In der zweiten Schaltsystemposition befinden sich die erste Innengewindenut und die zweite Innengewindenut nicht auf einer gemeinsamen Achse, sondern die Außengewindeabschnitte wirken form- und/oder kraftschlüssig mit den ersten Innengewindeabschnitten und/oder den zweiten Innengewindeabschnitten zusammen, so dass ein weiterführendes axiale Verschieben durch Druckausübung auf das Austragselement nicht mehr möglich ist. Ein weiterführendes Verbringen des Austragselements in das Behältnis, insbesondere zum Austragen des Knochenzements, wird durch ein Einschrauben des Austragselements in das Behältnis erreicht. Das Einschrauben kann soweit durchgeführt werden, bis die gewünschte Menge an Knochenzement appliziert wurde. Das Schaltsystems ist bevorzugt aus der ersten Schaltsystemposition in die zweite Schaltsystemposition zu verbringen, sobald das Austragselement so weit in das Behältnis eingeführt ist, dass das Austragen des Knochenzements beginnt. Zu diesem Zeitpunkt steigt der Kraftaufwand des Anwenders aufgrund der Viskosität des Knochenzements, wodurch ein Austragen durch Einschrauben für den Anwender leichter wird. Zudem erleichtert das Einschrauben die genaue Dosierung des Knochenzements im Vergleich zum Austragen mittels Einschieben. Das Einschieben in der zweiten Schaltsystemposition hat den Vorteil, dass es schnell erfolgen kann. Dies ist insbesondere während zeitkritischer Operationen und aufgrund des schnellen Aushärtens des Knochenzements, beispielsweise innerhalb von 5 Minuten, von Vorteil.

Die Vorrichtung ist dadurch gekennzeichnet, dass diese einen Knochenzement aus einem Behältnis austragen kann. Unter einem Knochenzement wird eine Substanz verstanden, die geeignet ist im Bereich der Medizintechnik eine stabile Verbindung zwischen künstlichen Gelenken, wie beispielsweise Hüft- und Kniegelenken, und Knochenmaterial zu erstellen. Bevorzugt handelt es sich bei Knochenzementen um Polymethylmethacrylat-Knochenzemente (PMMA Knochenzemente). PMMA-Knochenzemente kommen schon lange in medizinischen Anwendung zum Einsatz und gehen auf Arbeiten von Sir Charnley zurück (vgl. Charnley, J. Anchorage of the femoral head prosthesis of the shaft of the femur. J. Bone Joint Surg. 1960; 42, 28-30.). PMMA-Knochenzemente können dabei aus einem Knochenzementpulver als erster Ausgangskomponente und einer Monomerflüssigkeit als zweiter Ausgangskomponente hergestellt werden. Bei geeigneter Zusammensetzung können die beiden Ausgangskomponenten getrennt voneinander lagerstabil sein. Bei Inkontaktbringen der beiden Ausgangskomponenten entsteht durch Quellung der Polymerbestandteile des Knochenzementpulvers ein plastisch verformbarer Knochenzement, der auch als Knochenzementteig bezeichnet wird. Dabei wird eine Polymerisation des Monomers durch Radikale eingeleitet. Mit fortschreitender Polymerisation des Monomers erhöht sich die Viskosität des Knochenzements, bis dieser vollständig aushärtet.

Erfindungsgemäß wird unter einem Knochenzementpulver ein Pulver verstanden, welches mindestens ein partikuläres Polymethylmethacrylat und/oder ein partikuläres Polymethylmethacrylat-Copolymer umfasst. Beispiele für Copolymere sind Styren und/oder Methylacrylat. In einer Ausgestaltungsform kann das Knochenzementpulvers zusätzlich ein hydrophiles Additiv umfassen, welches die Verteilung der Monomerflüssigkeit innerhalb des Knochenzementpulvers unterstützt. In einer weiteren Ausgestaltungsform kann das Knochenzementpulver zusätzlich einen Initiator, welcher die Polymerisation einleitet, umfassen. In einer weiteren Ausgestaltungsform kann das Knochenzementpulvers zusätzlich einen Röntgenopaker umfassen. In noch einer weiteren Ausgestaltungsform kann das Knochenzementpulver zusätzlich pharmazeutisch aktive Substanzen, wie beispielsweise Antibiotika, umfassen.

Bevorzugt umfasst das Knochenzementpulver mindestens ein partikuläres Polymethylmethacrylat und/oder ein partikuläres Polymethylmethacrylat-Copolymer, einen Initiator und einen Röntgenopaker oder besteht aus diesen Komponenten. Weiter bevorzugt umfasst das Knochenzementpulver mindestens ein partikuläres Polymethylmethacrylat und/oder ein partikuläres Polymethylmethacrylat-Copolymer, einen Initiator, einen Röntgenopaker und ein hydrophiles Additiv oder besteht aus diesen Komponenten. Am bevorzugtesten umfasst das Knochenzementpulver mindestens ein partikuläres Polymethylmethacrylat und/oder ein partikuläres Polymethylmethacrylat-Copolymer, einen Initiator, einen Röntgenopaker, ein hydrophiles Additiv und ein Antibiotikum oder besteht aus diesen Komponenten.

Erfindungsgemäß kann die Partikelgröße des partikulären Polymethylmethacrylat und/oder des partikulären Polymethylmethacrylat-Copolymers des Knochenzementpulvers der Siebfraktion kleiner 150 µm, bevorzugt kleiner 100 µm, entsprechen.

Erfindungsgemäß kann das hydrophile Additiv partikulär und/oder faserförmig ausgestaltet sein. In einer weiteren Ausgestaltungsform kann das hydrophile Additiv schwerlöslich, bevorzugt unlöslich, in Methylmethacrylat sein. In einer weiteren Ausgestaltungsform kann das hydrophile Additiv ein Aufsaugvermögen von mindestens 0,6 g Methylmethacrylat pro Gramm hydrophiles Additiv besitzen. In einer weiteren Ausgestaltungsform kann das hydrophile Additiv eine chemische Substanz mit mindestens einer OH-Gruppe aufweisen. Dabei kann bevorzugt vorgesehen sein, dass das hydrophile Additiv kovalent gebundene OH-Gruppen an seiner Oberfläche besitzt. Beispiele für solche bevorzugten hydrophilen Additive können Additive ausgewählt aus der Gruppe umfassend Cellulose, Oxycellulose, Stärke, Titandioxid und Siliziumdioxid sein, wobei pyrogenes Siliziumdioxid besonders bevorzugt ist. In einer Ausgestaltungsform kann die Partikelgröße des hydrophilen Additivs der Siebfraktion kleiner 100 µm, bevorzugt kleiner 50 µm und am bevorzugtesten kleiner 10 µm entsprechen. Das hydrophile Additiv kann in einer Menge von 0,1 bis 2,5 Gew.-% bezogen auf das Gesamtgewicht des Knochenzementpulvers enthalten sein.

Erfindungsgemäß kann der Initiator Dibenzoylperoxid enthalten oder aus Dibenzoylperoxid bestehen.

Erfindungsgemäß versteht man unter einem Röntgenopaker eine Substanz, welche es erlaubt, den Knochenzement auf röntgendiagnostischen Aufnahmen sichtbar zu machen. Beispiele für Röntgenopaker können Bariumsulfat, Zirkondioxid und Kalziumcarbonat umfassen. Erfindungsgemäß kann die pharmazeutisch aktive Substanz ein oder mehrere Antibiotika und gegebenenfalls zugesetzte Co-Faktoren für das eine oder die mehreren Antibiotika umfassen. Bevorzugt besteht die pharmazeutisch aktive Substanz aus einem oder mehreren Antibiotika und gegebenenfalls zugesetzten Co-Faktoren für das eine oder die mehreren Antibiotika. Beispiele für Antibiotika sind unter anderem Gentamicin, Clindamycin und Vancomycin.

Erfindungsgemäß kann die Monomerflüssigkeit das Monomer Methylmethacrylat umfassen oder aus Methylmethacrylat bestehen. In einer Ausgestaltungsform umfasst die Monomerflüssigkeit neben dem Monomer einen darin gelösten Aktivator, wie beispielsweise N,N-Dimethyl-p-toluidin, oder besteht aus Methylmethacrylat und N,N-Dimethyl-p-toluidin.

### Beispiele

Die Erfindung wird im Folgenden durch Beispiele weiter beispielhaft illustriert. Die Erfindung ist nicht auf die Beispiele beschränkt.

### Figuren

Es zeigen
- Fig. 1: eine Vorrichtung zum Austragen eines Knochenzements aus einem Behältnis,
- Fig. 2: einen schematischen Querschnitt eines Schaltsystems der Vorrichtung aus Figur 1 in einer zweiten Schaltsystemposition,
- Fig. 3: einen schematischen Querschnitt des Schaltsystems der Vorrichtung aus Figur 2 in einer ersten Schaltsystemposition,
- Fig. 4: einen schematischen Querschnitt der Vorrichtung aus Figur 1 befestigt an einem mit einem Knochenzement befülltem Behältnis,
- Fig. 5: die Vorrichtung aus Figur 4 mit einem in das Behältnis eingeführtem Austragselement,
- Fig. 6: die Vorrichtung aus Figur 5 nach einem Austragen des Knochenzements aus dem Behältnis, und
- Fig. 7: ein Verfahren zum Austragen eines Knochenzements aus einem Behältnis mittels einer Vorrichtung.

### Beschreibung der Figuren

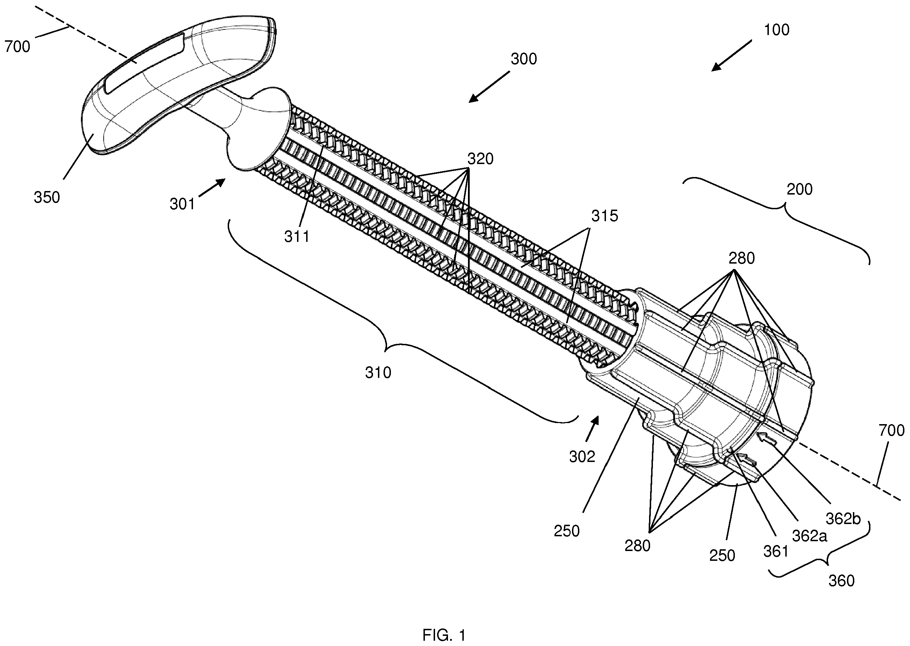

**Figur 1** zeigt eine Vorrichtung 100 zum Austragen eines Knochenzements. Die Vorrichtung 100 ist einstückig, aber aus mehreren Bauteilen aufgebaut. Die Vorrichtung weist ein Austragselement 300 auf. Das Austragselement 300 ist stangenartig ausgeformt und weist ein Außengewinde 310 auf. Das Außengewinde 310 ist durch axial verlaufende

Außengewindenuten 315 in Außengewindeabschnitte 320 unterteilt. Die Außengewindenuten 315 reduzieren eine Höhe einer Außengewindeflanke 311 des Außengewindes 310 auf Null. Die Außengewindenuten 315 weisen eine größere radiale Erstreckung als die Außengewindeabschnitte 320 auf. In einer weiteren, nicht gezeigten, Ausgestaltungsform weisen die Außengewindenuten 315 die gleiche radiale Erstreckung wie die Außengewindeabschnitte 320 auf.

An einem ersten Ende 301 weist das Austragselement 300 ein Schraubmittel 350 in Form eines Griffes auf. Das Schraubmittel 350 in Form eines Griffs erleichtert dem Anwender die Verwendung der Vorrichtung 100, insbesondere ein axiales Verschieben und ein Drehen des Austragselements 300 um eine Längsachse 700.

An einem zweiten Ende 302 weist die Vorrichtung 100 ein Schaltsystem 200 auf, wobei das Schaltsystem 200 manschettenartig um das Austragselement 300 angeordnet ist und axial entlang der gesamten Länge des Außengewindes 310 bewegbar ist. Das Schaltsystem 200 ist einstückig, aber aus mehreren Bauteilen aufgebaut. Das Schaltsystem 200 umfasst einen ersten Hohlzylinder 210 und einen zweiten Hohlzylinder 250, wobei der erste Hohlzylinder 210 und der zweite Hohlzylinder 250 axial miteinander verbunden sind. Der erste Hohlzylinder 210 und der zweite Hohlzylinder 250 sind rotatorisch um die Längsachse 700 mittels einer Drehbewegung gegeneinander bewegbar.

Um dem Anwender der Vorrichtung 100 die gegenläufige Drehbewegung des ersten Hohlzylinders 210 gegen den zweiten Hohlzylinder 250 zu erleichtern, weisen sowohl der erste Hohlzylinder 210 als auch der zweite Hohlzylinder 250 axial verlaufende Wülste 280 auf. Die Wülste 280 verbessern die Griffigkeit und erhöhen damit die Sicherheit gegen unerwünschtes Abrutschen im Zuge der Drehbewegung. In weiteren, nicht gezeigten, Ausgestaltungsformen weisen der erste Hohlzylinder 210 und der zweite Hohlzylinder 250 anstelle der Wülste 280 Noppen oder Rillen auf, um ein Abrutschen des Anwenders zu verhindern.

Durch die gegenläufige rotatorische Bewegung des ersten Hohlzylinders 210 gegen den zweiten Hohlzylinder 250 kann das Schaltsystem 200 reversibel zwischen einer ersten Schaltsystemposition und einer zweiten Schaltsystemposition verschoben werden. Um dem Anwender anzuzeigen, ob sich das Schaltsystem 200 in der ersten Schaltsystemposition oder in der zweiten Schaltsystemposition befindet, weist die Vorrichtung 100 optische Markierungen 360 in Form einer Einkerbung 361 am zweiten Hohlzylinder 250 und einem ersten Pfeil 361a und einem zweiten Pfeil 362b am ersten Hohlzylinder 210 auf. Liegen die Einkerbung 361 und der erste Pfeile 361a axial übereinander, zeigt dies dem Anwender die Einstellung des Schaltsystems 200 in der ersten Schaltsystemposition oder der zweiten Schaltsystemposition an. Ein axiales Übereinanderliegen der Einkerbung 361 mit dem zweiten Pfeil 361b zeigt dem Anwender die entsprechend andere Schaltsystemposition an. In weiteren, nicht gezeigten, Ausgestaltungsformen weist das Schaltsystem anstelle der Einkerbung 361 und dem ersten Pfeil 362a sowie dem zweiten Pfeil 362b andersartige optische Markierungen 360, wie beispielsweise Striche, Zahlen oder Ausbuchtungen auf.

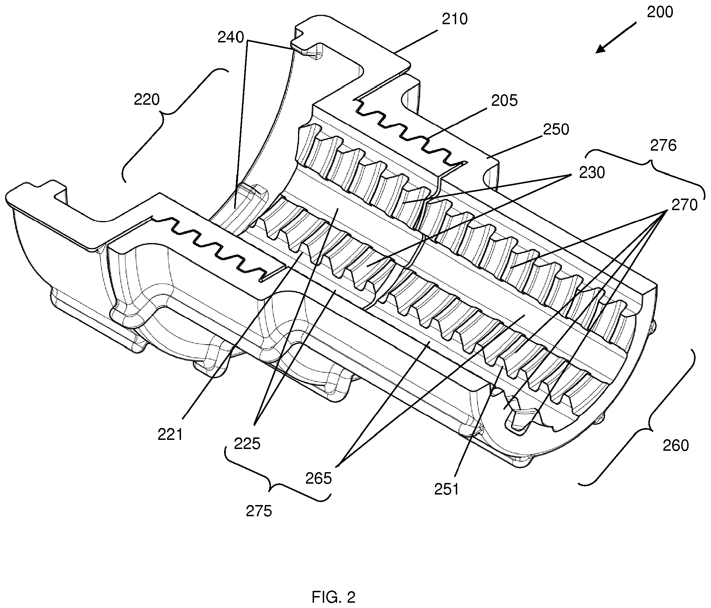

**Figur 2** zeigt einen Querschnitt des Schaltsystems 200 aus Figur 1. Der erste Hohlzylinder 210 und der zweite Hohlzylinder 250 sind über eine Gewindeverbindung 205 axial derart miteinander verbunden, dass eine gegenläufige Drehbewegung des ersten Hohlzylinders 210 gegen den zweiten Hohlzylinder 250 das Schaltsystem 200 reversibel zwischen der ersten Schaltsystemposition und der zweiten Schaltsystemposition verbringen kann.

Der erste Hohlzylinder 210 weist ein erstes Innengewinde 220 und der zweite Hohlzylinder 250 weist ein zweites Innengewinde 260 auf. Das erste Innengewinde 220 ist durch axial verlaufende erste Innengewindenuten 225 in erste Innengewindeabschnitte 230 unterteilt. Die ersten Innengewindenuten 225 reduzieren eine Höhe einer ersten Innengewindeflanke 221 auf Null. Das zweite Innengewinde 260 ist durch axial verlaufende zweite Innengewindenuten 265 in zweite Innengewindeabschnitte 270 unterteilt. Die zweiten Innengewindenuten 265 reduzieren eine Höhe einer zweiten Innengewindeflanke 251 auf Null. Die ersten Innengewindenuten 225 weisen eine größere radiale Erstreckung als die ersten Innengewindeabschnitte 230 und die zweiten Innengewindenuten 265 weisen eine größere radiale Erstreckung als die zweiten Innengewindeabschnitte 270 auf. Die ersten Innengewindenuten 225 und die zweiten Innengewindenuten 265 sowie die ersten Innengewindeabschnitte 230 und die zweiten Innengewindeabschnitte 270 weisen jeweils die gleiche radiale Erstreckung auf. In einer weiteren, nicht gezeigten Ausgestaltungsform, weisen die ersten Innengewindenuten 225, die ersten Innengewindeabschnitte 230, die zweiten Innengewindenuten 265 und die zweiten Innengewindeabschnitte 270 die gleiche radiale Erstreckung auf.

In Figur 2 ist das Schaltsystem 200 in der zweiten Schaltsystemposition gezeigt. In der zweiten Schaltsystemposition sind die ersten Innengewindenuten 225 und die zweiten Innengewindenuten 265 sowie die ersten Innengewindeabschnitte 230 und die zweiten Innengewindeabschnitte 270 entlang im Wesentlichen gemeinsam verlaufender Achsen angeordnet. Dadurch bilden die ersten Innengewindenuten 225 und die zweiten Innengewindenuten 265 gemeinsam über eine Gesamtlänge des ersten Innengewinde 210 und des zweiten Innengewinde 250 verlaufende Gesamtinnengewindenuten 275 sowie die ersten Innengewindeabschnitte 230 und die zweiten Innengewindeabschnitte 270 gemeinsam über die Gesamtlänge des ersten Innengewindes 210 und des zweiten Innengewindes 260 verlaufende Gesamtinnengewindeabschnitte 276.

Die Gesamtinnengewindenuten 275 weisen eine größere radiale Erstreckung als die Außengewindeabschnitte 320 in Figur 1 und die Gesamtinnengewindeabschnitte 276 weisen eine kleinere radiale Erstreckung als die Außengewindenuten 315 in Figur 1 auf. Dadurch ist das Austragselement 300 aus Figur 1 in der zweiten Schaltsystemposition mittels einer Schiebebewegung reversibel axial gegen das Schaltsystem 200 verschiebbar. Um das Austragselement 300 aus Figur 1 frei axial gegen das Schaltsystem 200 in der zweiten Schaltsystemposition verschieben zu können, sind die Außengewindeabschnitte 320 entlang der Gesamtinnengewindenuten 275 und die Außengewindenuten 315 entlang der Gesamtinnengewindeabschnitte 276 angeordnet.

Der erste Hohlzylinder weist ein Verbindungselement 240 in Form eines Bajonettverschlusses auf, mit dem die Vorrichtung 100 reversibel an einem entsprechenden Gegenstück eines Behältnisses, insbesondere an einem mit einem Knochenzement befüllten Behältnisses, befestigbar ist.

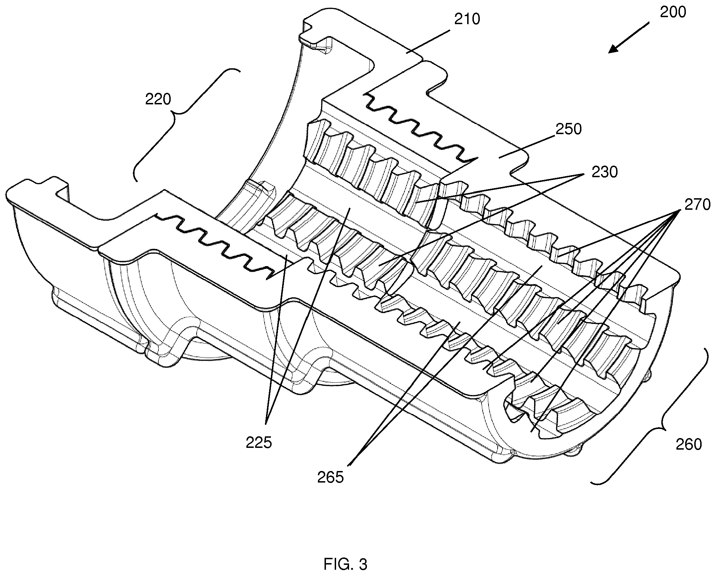

**Figur 3** zeigt das Schaltsystem 200 aus Figur 2 in der ersten Schaltsystemposition. Die Figur 2 und die Figur 3 zeigen das gleiche Schaltsystem 200, allerdings in verschiedenen Schaltsystempositionen. Figur 2 zeigt das Schaltsystem 200 in der zweiten Schaltsystemposition und Figur 3 zeigt das Schaltsystem 200 in der ersten Schaltsystemposition. Die ersten Innengewindenuten 225 und die zweiten Innengewindenuten 265, und damit auch die ersten Innengewindeabschnitte 230 und die zweiten Innengewindeabschnitte 270, sind jeweils nicht auf einer im Wesentlichen gemeinsamen Achse, sondern versetzt zueinander angeordnet. In axialer Erstreckung liegen die ersten Innengewindenuten 225 auf einer im Wesentlichen gemeinsamen Achse mit den zweiten Innengewindeabschnitten 270 und die ersten Innengewindeabschnitte 230 auf einer im Wesentlichen gemeinsamen Achse mit den zweiten Innengewindenuten 265. Damit ist es den Außengewindeabschnitten 320 aus Figur 1 in der ersten Schaltsystemposition nicht möglich, frei axial gegen das Schaltsystem 200 verschoben zu werden. In der ersten Schaltsystemposition sind das erste Innengewinde 200 und das zweite Innengewinde 250 derart zueinander angeordnet, dass die Außengewindeabschnitte 320 aus Figur 1 jederzeit form- und/oder kraftschlüssig mit den ersten Innengewindeabschnitten 230 und/oder den zweiten Innengewindeabschnitten 270 zusammenwirken, wodurch das Austragselement 300 aus Figur 1 ausschließlich durch eine Drehbewegung gegen das Schaltsystem 200 bewegbar ist.

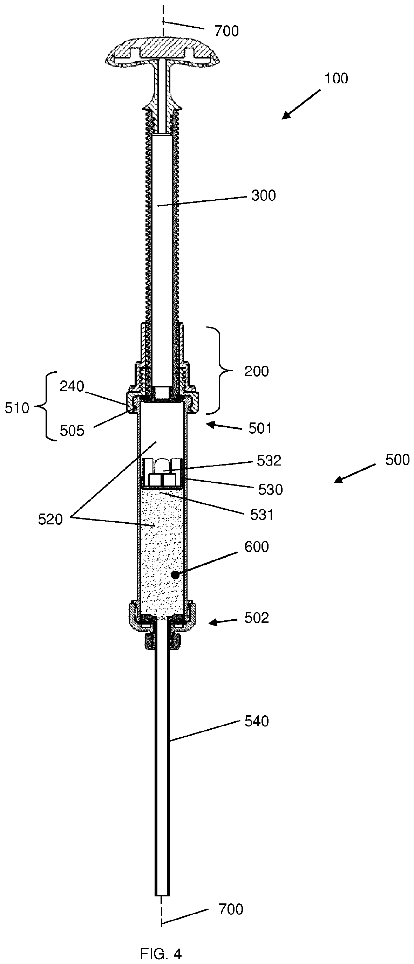

**Figur 4** zeigt die Vorrichtung 100 aus Figur 1 befestigt an einem ersten Behältnisende 501 eines Behältnisses 500. Die Vorrichtung 100 und das Behältnis 500 sind axial über das Verbindungselement 240 und einen Verbindungsanschluss 505 des Behältnisses 500 reversibel miteinander verbunden. Das Verbindungselement 240 und der Verbindungsanschluss 505 bilden zusammen einen Bajonettverschluss 510 aus. Die Vorrichtung 100 und das Behältnis 500 sind gemeinsam auf der Längsachse 700 angeordnet.

Das Behältnis 500 ist rohrartig ausgestaltet und weist in einem Innenraum 520 einen zur Austragung und zu Operationszwecken bereitstehenden Knochenzement 600 aus. Zum Austragen des Knochenzements 600 weist das Behältnis 500 im Innenraum 520 einen axial beweglichen Austragskolben 530 auf, welcher räumlich zwischen dem Austragselement 300 und dem Knochenzement 600 angeordnet ist. Der Austragskolben 530 weist eine dem Knochenzement 600 zugewandte flache Außenseite 531 mit einem Durchmesser, welcher im Wesentlichen einem Innendurchmesser des Behältnisses 500 entspricht und eine dem Austragselement 300 zugewandte Innenseite 532 auf, wobei die Innenseite 532 ausgestaltet ist, das Austragselement teilweise haubenartig aufzunehmen. In weiteren, nicht gezeigten, Ausgestaltungsformen ist die Außenseite 531 nicht flach, sondern beispielsweise konvex oder konisch geformt und/oder die Innenseite 532 ist nicht haubenartig, sondern beispielsweise flach ausgeformt. Um den Knochenzement 600 zielgerichtet an eine gewünschte Stelle zu applizieren, weist das Behältnis 500 an einem dem ersten Behältnisende 501 entgegengesetztem zweiten Behältnisende 502 einen Austragsschnorchel 540 auf.

Der Knochenzement 600 kann durch ein Einbringen des Austragselements 300 in das Behältnis 500 ausgetragen werden. Die Art des Einbringens des Austragselements 300 in das Behältnis 500 wird über das Schaltsystem 200 gesteuert. In der ersten Schaltsystemposition kann das Austragselements 300 ausschließlich mittels einer Drehbewegung in das Behältnis 500 eingebracht werden, während in der zweiten Schaltsystemposition das Austragselement 300 frei axial in das Behältnis 500 schiebbar ist. Die zweite Schaltsystemposition erlaubt somit ein schnelles und für den Anwender müheloses Einbringen des Austragselements 300, bevorzugt bis das Austragselement 300 in Kontakt mit dem Austragskolben 530 steht. Da der bereitgestellte Knochenzement 600 nur für kurze Zeit, beispielweise in einem Zeitfenster von bis zu 5 Minuten nach dem Bereitstellen, verwendbar ist, bevor das Aushärten zu weit fortgeschritten ist, ist die zweite Schaltsystemposition für das Einbringen des Austragselements 300 bis zum Kontakt mit dem Austragskolben 530 bevorzugt. In einer weiteren, nicht gezeigten Ausgestaltungsform, in der der Knochenzement 600 nicht bereits in Richtung des Austragsschnorchels 540 gesammelt im Behältnis 500 vorliegt, ist das Einbringen des Austragselements 300 in der zweiten Schaltsystemposition bevorzugt, bis der Knochenzement durch ein fortgeführtes Einbringen des Austragselements 300, und damit auch des Austragskolbens 530, den Knochenzement 600 in Richtung des Austragsschnorchels 540 verschoben und damit gesammelt hat und durch die pastöse Viskosität des Knochenzements 600 ein Austragen des Knochenzements 600 durch den Austragsschnorchel 540 mittels Schiebbewegung erschwert wird. Analoges gilt auch für weitere, nicht gezeigte, Ausgestaltungsformen ohne Austragskolben 530. In Ausgestaltungsformen ohne Austragskolben 530 ist das Austragselement 300 so ausgestaltet, dass zumindest ein dem Knochenzement 530 zugewandtes Ende einen Außendurchmesser aufweist, welcher im Wesentlichen einen Innendurchmesser des Behältnisses 500 entspricht, wodurch das Austragselement 300 selbst als Austragskolben wirkt.

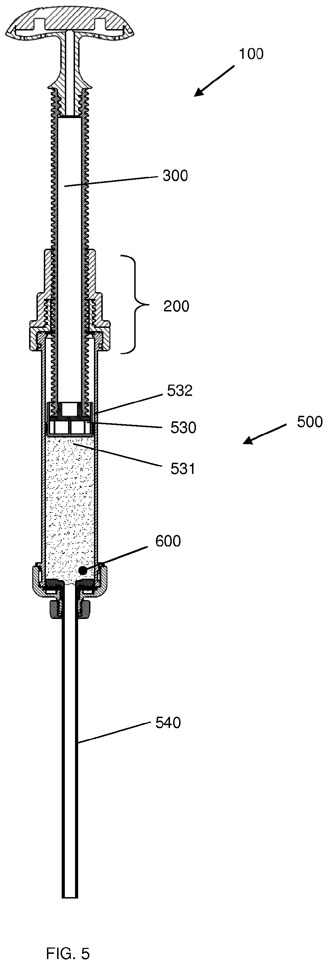

**Figur 5** zeigt die Vorrichtung 100 und das Behältnis 500 aus Figur 4 mit in das Behältnis 500 eingebrachtem Austragselement 300. Im Unterschied zu Figur 4, ist das Austragselement 300 in Kontakt mit dem Austragskolben 530, insbesondere mit der haubenartigen Innenseite 532 gebracht worden, wobei der Austragskolben 530 noch nicht in Richtung des Austragsschnorchels 540 verschoben wurde und damit das Austragen des Knochenzements 600 aus dem Behältnis 500 noch nicht eingesetzt hat. Ein fortgeführtes Einbringen des Austragselements 300 in das Behältnis 500 führt aufgrund der pastösen Viskosität und der im Vergleich zum Behältnis 500 verringerten Querschnittsfläche des Austragsschnorchels 540 zu einem erhöhten Kraftaufwand des Anwenders. Daher ist es bevorzugt, bei der in Figur 5 gezeigten Anordnung des Austragselements 300, das Schaltsystem 200 aus der zweiten Schaltsystemposition in die erste Schaltsystemposition zu verbringen. Dadurch ist das Austragselement 300 nicht mehr frei axial mittels Schiebbewegung in das Behältnis einschiebbar, sondern die Außengewindeabschnitte 320 aus Figur 1 wirken form- und/oder kraftschlüssig mit den ersten Innengewindeabschnitten 230 und/oder den zweiten Innengewindeabschnitten 270 aus Figur 3 zusammen. Dadurch ist das Austragselement 300 ausschließlich über eine Drehbewegung weiter in das Behältnis 500 einbringbar, was zum einen den benötigten Kraftaufwand beim Anwender reduziert und des Weiteren eine genauere Dosierung des Knochenzements 600 als bei einer schwer zu kontrollierenden Schiebbewegung ermöglicht.

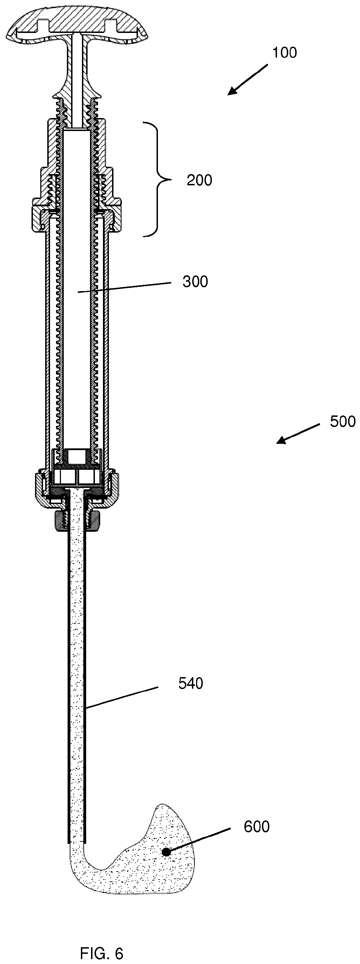

**Figur 6** zeigt die Vorrichtung 100 und das Behältnis 500 aus Figur 5 nach erfolgtem Austragen des Knochenzements 600. Das Austragselement 300 ist soweit wie möglich in das Behältnis 500 eingeführt, insbesondere eingedreht, worden. Der Knochenzement 600 ist vollständig aus dem Behältnis 500 an gewünschter Stelle ausgetragen worden, wobei der Austragsschnorchel 540 mit Resten des Knochenzements 600 gefüllt ist.

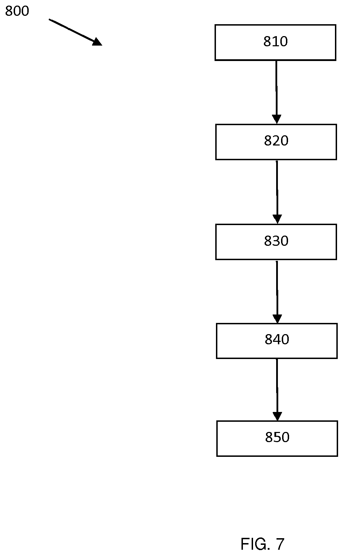

**Figur 7** zeigt ein Flussdiagramm enthalten die Schritte 810 bis 850 eines Verfahrens 800 zum Austragen eines Knochenzements 600 aus einem Behältnis 500 mittels der Vorrichtung 100 umfassend das Verbindungselement 240, um die Vorrichtung 100 mit dem Behältnis 500 reversibel zu verbinden, das stangenartige Austragselement 300, welches zumindest partiell das Außengewinde 310 aufweist, das Schaltsystem 200, welches zumindest partiell das erste Innengewinde 220 aufweist, wobei das erste Innengewinde 220 derart formschlüssig mit dem Außengewinde 310 koppelbar ist, dass in der ersten gekoppelten Schaltsystemposition das Austragselement 300 axial in das Behältnis 500 schraubbar ist und in der zweiten entkoppelten Schaltsystemposition das Austragselement 300 frei axial in das Behältnis 500 schiebbar ist. In einer bevorzugten Ausgestaltungsform ist innerhalbe des Behältnisses 500 der Austragskolben 530 zwischen dem Austragselement 300 und dem Knochenzement 600 gelagert.

In einem Schritt 810 wird die Vorrichtung 100 reversibel axial mit dem Behältnis 500 mittels des Verbindungselements 240 verbunden. Bevorzugt bildet das Verbindungselement 240 mit dem Behältnis 500 den Bajonettverschluss 510 aus, um die Vorrichtung 100 reversibel mit dem Behältnis 500 zu verbinden.

In einem weiteren Schritt 820 wird das Schaltsystem 200 in die zweite Schaltsystemposition verbracht, damit das Austragselement frei axial in das Behältnis 500 einschiebbar ist. In einer Ausgestaltungsform des Verfahrens 800 findet das Verbinden der Vorrichtung 100 mit dem Behältnis 500 in Schritt 810 zeitlich vor dem Schritt 820 statt. In einer weiteren Ausgestaltungsform des Verfahrens 800 wurde das Schaltsystem 200 vor dem Schritt 810 in die zweite Schaltsystemposition verbracht.

In einem weiteren Schritt 830 wird das Austragselement 300 frei axial in das Behältnis 500 eingeschoben. Bevorzugt wird das Austragselement 300 so weit in das Behältnis eingeschoben, bis der Anwender einen deutlichen Widerstand gegen die Schiebbewegung erfährt. Dieser kann beispielsweise bei Kontakt mit dem Austragskolben 530 oder mit beginnendem Verbringen des Knochenzements aus dem Behältnis 500 in den Austragsschnorchel 540 auftreten. Ein Vorteil des freien axialen Einschiebens des Austragselements 300 in das Behältnis 500 ist die schnelle Ausführbarkeit des Schrittes 820.

Dies ist gerade im Zuge zeitkritischer Operation und da der Knochenzeit 600 vor dem Aushärten in Abhängigkeit der chemischen Zusammensetzung nur eine kurze Zeitspanne, beispielsweise bis zu 5 Minuten, zum Verbringen an die gewünschte Stelle zur Verfügung steht, von Bedeutung.

Ist das Austragelement 300 bis an die gewünschte Stelle in das Behältnis 500 eingeschoben, wird in einem weiteren Schritt 840 das Schaltsystem 200 durch eine rotatorische Bewegung um die Längsachse 700 des Austragselements in die erste Schaltsystemposition verbracht. In der ersten Schaltsystemposition ist das Austragselement 300 nicht mehr frei axial in das Behältnis einschiebbar, sondern kann nur noch über eine Drehbewegung weiter eingebracht werden. Das Verbringen des Schaltsystems 200 aus der zweiten Schaltsystemposition in die erste Schaltsystemposition ist durch eine der rotatorischen Bewegung entgegengesetzte weitere rotatorischen Bewegung möglich. Ein Vorteil des Verbringens des Schaltsystems 200 aus der zweiten Schaltsystemposition in die erste Schaltsystemposition durch eine rotatorische Bewegung ist, dass der Anwender Vorrichtung 100 nach erfolgtem Umschalten wieder beide Hände zum weiteren Bedienen der Vorrichtung 100 oder weiterer Operationsgegenstände zur Verfügung hat. Das Schaltsystem 200 verbleibt selbstständig in der gewählten Schaltsystemposition, bis der Anwender wieder selbst aktiv wird.

In einer bevorzugten Ausgestaltungsform des Verfahrens 800 erfolgt das reversible Verbringen des Schaltsystem 200 in die erste Schaltsystemposition und die zweite Schaltsystemposition durch eine gegenläufige rotatorische Bewegung des ersten Hohlzylinders 210 gegen den zweiten Hohlzylinder 250 um die Längsachse 700 des Austragselements 300. Die gegenläufige rotatorische Bewegung verbringt die zumindest eine entlang der Längsachse verlaufende erste Innengewindenut 225 des ersten Innengewindes 220 und die zumindest eine zweite Innengewindenut 265 des zweiten Innengewindes 260 auf eine gemeinsame Achse. Die zumindest eine erste Innengewindenut 225 und die zumindest eine zweite Innengewindenut 265 formen in der zweiten Schaltsystem die im Wesentlichen geradlinig verlaufende Gesamtinnengewindenut 275. Sind die Außengewindeabschnitte 320 des Außengewindes 310 entlang der im Wesentlichen gleichen Achse wie die Gesamtinnengewindenut 275 angeordnet, ist das Austragselement 300 in Schritt 830 frei axial in das Behältnis 500 einschiebbar. Eine weitere rotatorische Bewegung in die erste Schaltsystemposition verschiebt die erste Innengewindenut 225 gegen die zweite Innengewindenut 265 aus der im Wesentlichen gemeinsamen Achse heraus, wodurch die Außengewindeabschnitte 320 nicht mehr frei axial verschiebbar sind, sondern stattdessen mit den ersten Innengewindeabschnitten 230 und/oder den zweiten Innengewindeabschnitten 270 form- und/oder kraftschlüssig zusammenwirken.

Dies ermöglicht in der ersten Schaltsystemposition ein Einschrauben des Austragselements 300 in das Behältnis 500.

In einem weiteren Schritt 850 wird der Knochenzement 600 aus dem Behältnis 500 durch ein Einschrauben des Austragselements 300 in das Behältnis 500 ausgetragen. Dabei wird das Austragselement 300 so weit in das Behältnis 500 eingedreht, bis die gewünschte Menge an Knochenzement 600 appliziert wurde. Zudem erleichtert das Eindrehen dem Anwender das Austragen des pastösen Knochenzements 600 im Vergleich zum Einschieben des Austragselements 300.

Die in den Ansprüchen, der Beschreibung und in den Figuren offenbarten Merkmale können für verschiedene Ausgestaltungsformen der beanspruchten Erfindung sowohl getrennt als auch in beliebiger Kombination miteinander wesentlich sein. Die für die Vorrichtung offenbarten Merkmale sind auch für das Verfahren offenbart und umgekehrt.

### Bezugszeichen

- 100: Vorrichtung
- 200: Schaltsystem
- 205: Gewindeverbindung
- 210: erster Hohlzylinder
- 220: erstes Innengewinde
- 221: erste Innengewindeflanke
- 225: erste Innengewindenut
- 230: erste Innengewindeabschnitte
- 240: Verbindungselement
- 250: zweiter Hohlzylinder
- 251: zweite Innengewindeflanke
- 260: zweites Innengewinde
- 265: zweite Innengewindenut
- 270: zweite Innengewindeabschnitte
- 275: Gesamtinnengewindenut
- 276: Gesamtinnengewindeabschnitt
- 280: Wulst
- 300: Austragselement
- 301: erstes Ende des Austragselements
- 302: zweites Ende des Austragselements
- 310: Außengewinde
- 311: Außengewindeflanke
- 301: erstes Ende des Austragselements
- 302: zweites Ende des Austragselements
- 311: Außengewindeflanke
- 315: Außengewindenut
- 320: Außengewindeabschnitte
- 350: Schraubmittel
- 360: optische Markierungen
- 361: Einkerbung
- 362a: erster Pfeil
- 362b: zweiter Pfeil
- 500: Behältnis
- 501: erstes Behältnisende
- 502: zweites Behältnisende
- 505: Verbindungsanschluss
- 510: Bajonettverschluss
- 520: Innenraum
- 530: Austragskolben
- 531: Außenseite
- 532: Innenseite
- 540: Austragsschnorchel
- 600: Knochenzement
- 700: Längsachse
- 800: Verfahren zum Austragen von Knochenzement
- 810: Verbinden der Vorrichtung mit dem Behältnis
- 820: Verbringen des Schaltsystems in die zweite Schaltsystemposition
- 830: axiales Verschieben des Austragselements in das Behältnis
- 840: Verbringen des Schaltsystems in die zweite Schaltsystemposition
- 850: Austragen des Knochenzements

## Patentansprüche

1. Vorrichtung (100) zum Austragen eines Knochenzements (600) aus einem Behältnis (500), umfassend
ein Verbindungselement (240), um die Vorrichtung (100) mit dem Behältnis (500) reversibel zu verbinden,
ein stangenartiges Austragselement (300), welches zumindest partiell ein Außengewinde (310) aufweist,
ein Schaltsystem (200), welches zumindest partiell ein erstes Innengewinde (220) aufweist,
wobei das erste Innengewinde (220) derart formschlüssig mit dem Außengewinde (310) koppelbar ist, dass
in einer ersten gekoppelten Schaltsystemposition das Austragselement (300) axial in das Behältnis (500) schraubbar ist und
in einer zweiten entkoppelten Schaltsystemposition das Austragselement (300) frei axial in das Behältnis (500) schiebbar ist,
**dadurch gekennzeichnet, dass**
das erste Innengewinde (220) das Austragselement (300) manschettenartig umfasst und das Schaltsystem (200) mittels einer rotatorischen Bewegung um eine Längsachse (700) des Austragselements (300) reversibel zwischen der ersten Schaltsystemposition und der zweiten Schaltsystemposition verschiebbar ist.

2. Vorrichtung (100) nach Anspruch 1, **dadurch gekennzeichnet, dass** das Schaltsystem (200) einen ersten Hohlzylinder (210) aufweist, welcher das erste Innengewinde (220), und einen, mit dem ersten Hohlzylinder (210) axial verbundenen, zweiten Hohlzylinder (250) aufweist,
wobei der erste Hohlzylinder (210) und der zweite Hohlzylinder (250) derart verbunden sind, dass das Schaltsystem (200) mittels einer gegenläufigen rotatorischen Bewegung des ersten Hohlzylinders (210) gegen den zweiten Hohlzylinder (250) um die Längsachse (700) reversibel zwischen der ersten Schaltsystemposition und der zweiten Schaltsystemposition verschiebbar ist.

3. Vorrichtung (100) nach Anspruch 2, **dadurch gekennzeichnet, dass** der zweite Hohlzylinder (250) zumindest partiell ein zweites Innengewinde (260) aufweist und das Außengewinde (310) zumindest eine entlang der Längsachse (700) verlaufende Außengewindenut (315),
das erste Innengewinde (220) zumindest eine entlang der Längsachse (700) verlaufende erste Innengewindenut (225) und
das zweite Innengewinde (260) zumindest eine entlang der Längsachse (700) verlaufende zweite Innengewindenut (265) aufweist.

4. Vorrichtung (100) nach Anspruch 3, **dadurch gekennzeichnet, dass** die zumindest eine Außengewindenut (315) das Außengewinde (310) in Außengewindeabschnitte (320),
die zumindest eine erste Innengewindenut (225) das erste Innengewinde (220) in erste Innengewindeabschnitte (230) und die zumindest eine zweite Innengewindenut (265) das zweite Innengewinde (260) in zweite Innengewindeabschnitte (270) entlang der Längsachse (700) unterteilt.

5. Vorrichtung (100) nach Anspruch 4, **dadurch gekennzeichnet, dass** eine radiale Erstreckung der zumindest einen Außengewindenut (315), der zumindest einen ersten Innengewindenut (225) und der zumindest einen zweiten Innengewindenut (265) gleich oder größer einer radialen Erstreckung des Außengewindeabschnittes (320), des ersten Innengewindeabschnittes (230) und des zweiten Innengewindeabschnittes (270) ist.

6. Vorrichtung (100) nach einem der Ansprüche 3 bis 5, **dadurch gekennzeichnet, dass** das Außengewinde (310), das erste Innengewinde (220) und das zweite Innengewinde (250) einen im Wesentlichen gleichen Steigungswinkel aufweisen.

7. Vorrichtung (100) nach einem der Ansprüche 3 bis 6, **dadurch gekennzeichnet, dass** das Außengewinde (310), das erste Innengewinde (220) und das zweite Innengewinde (260) jeweils eine gleiche Anzahl von Außengewindenuten (325), ersten Innengewindenuten (225) und zweiten Innengewindenuten (270) mit der jeweils im Wesentlichen gleichen radialen Erstreckung aufweist.

8. Vorrichtung (100) nach einem der Ansprüche 3 bis 7, **dadurch gekennzeichnet, dass** in der zweiten Schaltsystemposition die zumindest eine erste Innengewindenut (225) und die zumindest eine zweite Innengewindenut (265) auf einer im Wesentlichen gemeinsamen Achse liegen.

9. Vorrichtung (100) nach Anspruch 8, **dadurch gekennzeichnet, dass** in der zweiten Schaltsystemposition die zumindest eine erste Innengewindenut (225) und die zumindest eine zweite Innengewindenut (265) derart zu dem Außengewinde (310) ausgerichtet sind, insbesondere auf einer gemeinsamen Achse liegen, dass das Außengewinde (310) axial frei in die erste Innengewindenut (225) und die zweite Innengewindenut (265) schiebbar ist, um das Austragselement (300) frei axial in das Behältnis (500) zu schieben.

10. Vorrichtung (100) nach einem der Ansprüche 3 bis 9, **dadurch gekennzeichnet, dass** in der ersten Schaltsystemposition die zumindest eine erste Innengewindenut (225) und die zumindest eine zweiten Innengewindenut (265) nicht auf einer gemeinsamen Achse liegen.

11. Vorrichtung (100) nach Anspruch 10, **dadurch gekennzeichnet, dass** in der ersten Schaltsystemposition die zumindest eine erste Innengewindenut (225) und die zumindest eine zweite Innengewindenut (265) nicht auf einer gemeinsamen Achse liegen, so dass das Außengewinde (310) mit dem ersten Innengewinde (220) und dem zweiten Innengewinde (260) derart miteinander koppelt, dass das Austragselement (300) axial in das Behältnis (500) schraubbar ist.

12. Vorrichtung (100) nach einem der vorhergehenden Ansprüche, **dadurch gekennzeichnet, dass** die Vorrichtung (100) aus Kunststoff, insbesondere aus einem kompostierbaren Kunststoff, bevorzugt aus einem Glasfaser-verstärktem Kunststoff, Metall oder aus einer Kombination von Kunststoff, insbesondere aus einem kompostierbaren Kunststoff, bevorzugt aus einem Glasfaser-verstärktem Kunststoff und Metall gefertigt ist.

13. Vorrichtung (100) nach einem der Ansprüche 2 bis 12, **dadurch gekennzeichnet, dass** der erste Hohlzylinder (210) das Verbindungselement (240) umfasst.

14. Verfahren (800) zum Austragen eines Knochenzements (600) aus einem Behältnis (500) mit einer Vorrichtung (100) umfassend
ein Verbindungselement (240), um die Vorrichtung (100) mit dem Behältnis (500) reversibel zu verbinden,
ein stangenartiges Austragselement (300), welches zumindest partiell ein Außengewinde (310) aufweist,
ein Schaltsystem (200), welches zumindest partiell ein erstes Innengewinde (220) aufweist,
wobei das erste Innengewinde (220) derart formschlüssig mit dem Außengewinde (310) koppelbar ist, dass
in einer ersten gekoppelten Schaltsystemposition das Austragselement (300) axial in das Behältnis (500) schraubbar ist und
in einer zweiten entkoppelten Schaltsystemposition das Austragselement (300) frei axial in das Behältnis (500) schiebbar ist,
**zumindest umfassend die Schritte**
a) Verbinden der Vorrichtung (100) mittels des Verbindungselements (240) an dem Behältnis (500);
b) Verbringen des Schaltsystems (200) in die zweite Schaltsystemposition;
c) axiales Verschieben des Austragselements (300) in das Behältnis (500);
d) Verbringen des Schaltsystems (200) in die erste Schaltsystemposition durch eine rotatorische Bewegung um eine Längsachse des Austragselements (300);
e) Austragen des Knochenzements (500) aus dem Behältnis (500) durch ein Einschrauben des Austragselements (300) in das Behältnis (500).

15. Verfahren (800) nach Anspruch 14, **dadurch gekennzeichnet, dass** die rotatorische Bewegung in Schritt d) einen ersten Hohlzylinder (220) des Schaltsystems (200) gegen einen zweiten Hohlzylinder (250) des Schaltsystems (200) rotatorisch verschiebt.

16. Verfahren (800) nach Anspruch 14 oder 15, **dadurch gekennzeichnet, dass** der erste Hohlzylinder (210) das erste Innengewinde (220) und der zweite Hohlzylinder (250) ein zweites Innengewinde (260) aufweist,
wobei das Außengewinde (310) zumindest eine Außengewindenut (315),
das erste Innengewinde (220) zumindest eine erste Innengewindenut (225) und
das zweite Innengewinde (260) zumindest eine zweite Innengewindenut (265) umfassen, und
die zumindest eine Außengewindenut (315) das Außengewinde (310) in Außengewindeabschnitte (320),
die zumindest eine erste Innengewindenut (225) das erste Innengewinde (220) in erste Innengewindeabschnitte (230) und
die zumindest eine zweite Innengewindenut (265) das zweite Innengewinde (260) in zweite Innengewindeabschnitte (270) jeweils entlang der Längsachse (700) unterteilt und
wobei die zumindest eine erste Innengewindenut (225) und die zumindest eine zweite Innengewindenut (265) in der zweiten Schaltsystemposition auf einer im Wesentlichen gemeinsamen Achse angeordnet sind, so dass in Schritt c) die Außengewindeabschnitte (320) entlang der gemeinsamen Achse in das Behältnis (500) verschoben werden.

## Claims

1. A device (100) for dispensing a bone cement (600) from a container (500), comprising a connecting element (240) for reversibly connecting the device (100) to the container (500),
a rod-like dispensing element (300) which at least partially comprises an external thread (310),
a switching system (200) which at least partially comprises a first internal thread (220),
it being possible for the first internal thread (220) to be coupled form-fittingly to the external thread (310) such that
in a first coupled switching system position, the dispensing element (300) can be screwed axially into the container (500), and
in a second decoupled switching system position, the dispensing element (300) can be freely pushed axially into the container (500),
**characterized in that**
the first internal thread (220) encompasses the dispensing element (300) in a sleeve-like manner and the switching system (200) can be reversibly shifted between the first switching system position and the second switching system position by means of a rotational movement about a longitudinal axis (700) of the dispensing element (300).

2. The device (100) according to claim 1, **characterized in that** the switching system (200) comprises a first hollow cylinder (210), which comprises the first internal thread (220), and a second hollow cylinder (250), which is axially connected to the first hollow cylinder (210),
the first hollow cylinder (210) and the second hollow cylinder (250) being connected such that the switching system (200) can be reversibly shifted between the first switching system position and the second switching system position by means of a rotational movement of the first hollow cylinder (210) in the opposite direction to the second hollow cylinder (250) about the longitudinal axis (700).

3. The device (100) according to claim 2, **characterized in that** the second hollow cylinder (250) at least partially comprises a second internal thread (260), and the external thread (310) comprises at least one external thread groove (315) which extends along the longitudinal axis (700),
the first internal thread (220) comprises at least one first internal thread groove (225) which extends along the longitudinal axis (700), and
the second internal thread (260) comprises at least one second internal thread groove (265) which extends along the longitudinal axis (700).

4. The device (100) according to claim 3, **characterized in that** the at least one external thread groove (315) divides the external thread (310) into external thread portions (320), the at least one first internal thread groove (225) divides the first internal thread (220) into first internal thread portions (230) and the at least one second internal thread groove (265) divides the second internal thread (260) into second internal thread portions (270) along the longitudinal axis (700).

5. The device (100) according to claim 4, **characterized in that** a radial extension of the at least one external thread groove (315), the at least one first internal thread groove (225) and the at least one second internal thread groove (265) is equal to or greater than a radial extension of the external thread portion (320), the first internal thread portion (230) and the second internal thread portion (270).

6. The device (100) according to any of claims 3 to 5, **characterized in that** the external thread (310), the first internal thread (220) and the second internal thread (250) have a substantially identical angle of inclination.

7. The device (100) according to any of claims 3 to 6, **characterized in that** the external thread (310), the first internal thread (220) and the second internal thread (260) each comprise an identical number of external thread grooves (325), first internal thread grooves (225) and second internal thread grooves (270), each having the substantially identical radial extension.

8. The device (100) according to any of claims 3 to 7, **characterized in that** the at least one first internal thread groove (225) and the at least one second internal thread groove (265) are on a substantially common axis in the second switching system position.

9. The device (100) according to claim 8, **characterized in that** the at least one first internal thread groove (225) and the at least one second internal thread groove (265) are oriented relative to the external thread (310), in particular are on a common axis, in the second switching system position such that the external thread (310) can be freely pushed axially into the first internal thread groove (225) and the second internal thread groove (265) in order to freely push the dispensing element (300) axially into the container (500).

10. The device (100) according to any of claims 3 to 9, **characterized in that** the at least one first internal thread groove (225) and the at least one second internal thread groove (265) are not on a common axis in the first switching system position.

11. The device (100) according to claim 10, **characterized in that** the at least one first internal thread groove (225) and the at least one second internal thread groove (265) are not on a common axis in the first switching system position such that the external thread (310) couples with the first internal thread (220) and the second internal thread (260) such that the dispensing element (300) can be screwed axially into the container (500).

12. The device (100) according to any of the preceding claims, **characterized in that** the device (100) is made of plastics material, in particular a compostable plastics material, preferably a glass fiber-reinforced plastics material, of metal, or of a combination of plastics material, in particular a compostable plastics material, preferably a glass fiber-reinforced plastics material, and metal.

13. The device (100) according to any of claims 2 to 12, **characterized in that** the first hollow cylinder (210) encompasses the connecting element (240).

14. A method (800) for dispensing a bone cement (600) from a container (500) using a device (100) comprising
a connecting element (240) for reversibly connecting the device (100) to the container (500),
a rod-like dispensing element (300) which at least partially comprises an external thread (310),
a switching system (200) which at least partially comprises a first internal thread (220),
it being possible for the first internal thread (220) to be coupled form-fittingly to the external thread (310) such that
in a first coupled switching system position, the dispensing element (300) can be screwed axially into the container (500), and
in a second decoupled switching system position, the dispensing element (300) can be freely pushed axially into the container (500),
**at least comprising the steps of**
a) connecting the device (100) to the container (500) by means of the connecting element (240);
b) moving the switching system (200) into the second switching system position;
c) axially shifting the dispensing element (300) into the container (500);
d) moving the switching system (200) into the first switching system position by means of a rotational movement about a longitudinal axis of the dispensing element (300);
e) dispensing the bone cement (500) from the container (500) by screwing the dispensing element (300) into the container (500).

15. The method (800) according to claim 14, **characterized in that** the rotational movement in step d) rotationally shifts a first hollow cylinder (220) of the switching system (200) against a second hollow cylinder (250) of the switching system (200).

16. The method (800) according to either claim 14 or claim 15, **characterized in that** the first hollow cylinder (210) comprises the first internal thread (220) and the second hollow cylinder (250) comprises a second internal thread (260),
the external thread (310) comprising at least one external thread groove (315), the first internal thread (220) comprising at least one first internal thread groove (225) and the second internal thread (260) comprising at least one second internal thread groove (265), and
the at least one external thread groove (315) dividing the external thread (310) into external thread portions (320),
the at least one first internal thread groove (225) dividing the first internal thread (220) into first internal thread portions (230), and
the at least one second internal thread groove (265) dividing the second internal thread (260) into second internal thread portions (270) in each case along the longitudinal axis (700), and
the at least one first internal thread groove (225) and the at least one second internal thread groove (265) being arranged on a substantially common axis in the second switching system position so that, in step c), the external thread portions (320) are shifted along the common axis into the container (500).

## Revendications

1. Dispositif (100) permettant l'application d'un ciment osseux (600) à partir d'un récipient (500), comprenant
un élément de liaison (240) pour relier de manière réversible le dispositif (100) au récipient (500),
un élément d'application (300) en forme de tige, lequel présente au moins partiellement un filetage extérieur (310),
un système de commutation (200), lequel présente au moins partiellement un premier filetage intérieur (220),
dans lequel le premier filetage intérieur (220) peut être accouplé par complémentarité de forme au filetage extérieur (310) de telle sorte que
dans une première position de système de commutation accouplée, l'élément d'application (300) peut être vissé axialement dans le récipient (500) et
dans une seconde position de système de commutation désaccouplée, l'élément d'application (300) peut être déplacé librement de manière axiale dans le récipient (500), **caractérisé en ce que**
le premier filetage intérieur (220) entoure l'élément d'application (300) à la manière d'un manchon et le système de commutation (200) peut être déplacé de manière réversible, à l'aide d'un mouvement de rotation autour d'un axe longitudinal (700) de l'élément d'application (300), entre la première position de système de commutation et la seconde position de système de commutation.

2. Dispositif (100) selon la revendication 1, **caractérisé en ce que** le système de commutation (200) présente un premier cylindre creux (210), lequel présente le premier filetage intérieur (220), et un second cylindre creux (250) relié axialement au premier cylindre creux (210),
dans lequel le premier cylindre creux (210) et le second cylindre creux (250) sont reliés de telle sorte que le système de commutation (200) peut être déplacé de manière réversible, à l'aide d'un mouvement de rotation opposé du premier cylindre creux (210) contre le second cylindre creux (250) autour de l'axe longitudinal (700), entre la première position de système de commutation et la seconde position de système de commutation.

3. Dispositif (100) selon la revendication 2, **caractérisé en ce que** le second cylindre creux (250) présente au moins partiellement un second filetage intérieur (260) et le filetage extérieur (310) présente au moins une rainure de filetage extérieur (315) s'étendant le long de l'axe longitudinal (700),
le premier filetage intérieur (220) présente au moins une première rainure de filetage intérieur (225) s'étendant le long de l'axe longitudinal (700) et
le second filetage intérieur (260) présente au moins une seconde rainure de filetage intérieur (265) s'étendant le long de l'axe longitudinal (700).

4. Dispositif (100) selon la revendication 3, **caractérisé en ce que,** le long de l'axe longitudinal (700), l'au moins une rainure de filetage extérieur (315) divise le filetage extérieur (310) en sections de filetage extérieur (320),
l'au moins une première rainure de filetage intérieur (225) divise le premier filetage intérieur (220) en premières sections de filetage intérieur (230) et l'au moins une seconde rainure de filetage intérieur (265) divise le second filetage intérieur (260) en secondes sections de filetage intérieur (270).

5. Dispositif (100) selon la revendication 4, **caractérisé en ce qu'**une extension radiale de l'au moins une rainure de filetage extérieur (315), de l'au moins une première rainure de filetage intérieur (225) et de l'au moins une seconde rainure de filetage intérieur (265) est supérieure ou égale à une extension radiale de la section de filetage extérieur (320), de la première section de filetage intérieur (230) et de la seconde section de filetage intérieur (270).

6. Dispositif (100) selon l'une des revendications 3 à 5, **caractérisé en ce que** le filetage extérieur (310), le premier filetage intérieur (220) et le second filetage intérieur (250) présentent un angle d'inclinaison sensiblement égal.

7. Dispositif (100) selon l'une des revendications 3 à 6, **caractérisé en ce que** le filetage extérieur (310), le premier filetage intérieur (220) et le second filetage intérieur (260) présentent respectivement un nombre égal de rainures de filetage extérieur (325), de premières rainures de filetage intérieur (225) et de secondes rainures de filetage intérieur (270) avec l'extension radiale respectivement sensiblement égale.

8. Dispositif (100) selon l'une des revendications 3 à 7, **caractérisé en ce que,** dans la seconde position de système de commutation, l'au moins une première rainure de filetage intérieur (225) et l'au moins une seconde rainure de filetage intérieur (265) se trouvent sur un axe sensiblement commun.

9. Dispositif (100) selon la revendication 8, **caractérisé en ce que,** dans la seconde position de système de commutation, l'au moins une première rainure de filetage intérieur (225) et l'au moins une seconde rainure de filetage intérieur (265) sont alignées avec le filetage extérieur (310), en particulier se trouvent sur un axe commun, de telle sorte que le filetage extérieur (310) peut être déplacé librement de manière axiale dans la première rainure de filetage intérieur (225) et la seconde rainure de filetage intérieur (265) afin de déplacer librement et de manière axiale l'élément d'application (300) dans le récipient (500).

10. Dispositif (100) selon l'une des revendications 3 à 9, **caractérisé en ce que,** dans la première position de système de commutation, l'au moins une première rainure de filetage intérieur (225) et l'au moins une seconde rainure de filetage intérieur (265) ne se trouvent pas sur un axe commun.

11. Dispositif (100) selon la revendication 10, **caractérisé en ce que,** dans la première position de système de commutation, l'au moins une première rainure de filetage intérieur (225) et l'au moins une seconde rainure de filetage intérieur (265) ne se trouvent pas sur un axe commun, de sorte que le filetage extérieur (310) est accouplé au premier filetage intérieur (220) et au second filetage intérieur (260) de telle sorte que l'élément d'application (300) peut être vissé axialement dans le récipient (500).

12. Dispositif (100) selon l'une des revendications précédentes, **caractérisé en ce que** le dispositif (100) est fabriqué en matière plastique, en particulier en une matière plastique compostable, préférablement en une matière plastique renforcée de fibres de verre, un métal ou en une combinaison de matière plastique, en particulier en une matière plastique compostable, préférablement en une matière plastique renforcée de fibres de verre et un métal.

13. Dispositif (100) selon l'une des revendications 2 à 12, **caractérisé en ce que** le premier cylindre creux (210) comprend l'élément de liaison (240).

14. Procédé (800) permettant l'application d'un ciment osseux (600) à partir d'un récipient (500), comportant un dispositif (100) comprenant
un élément de liaison (240) pour relier de manière réversible le dispositif (100) au récipient (500),
un élément d'application (300) en forme de tige, lequel présente au moins partiellement un filetage extérieur (310),
un système de commutation (200), lequel présente au moins partiellement un premier filetage intérieur (220),
dans lequel le premier filetage intérieur (220) peut être accouplé par complémentarité de forme au filetage extérieur (310) de telle sorte que
dans une première position de système de commutation accouplée, l'élément d'application (300) peut être vissé axialement dans le récipient (500) et
dans une seconde position de système de commutation désaccouplée, l'élément d'application (300) peut être déplacé librement de manière axiale dans le récipient (500), **comprenant au moins les étapes consistant à**
a) relier le dispositif (100) au récipient (500) à l'aide de l'élément de liaison (240) ;
b) amener le système de commutation (200) dans la seconde position de système de commutation ;
c) déplacer axialement l'élément d'application (300) dans le récipient (500) ;
d) amener le système de commutation (200) dans la première position de système de commutation par un mouvement de rotation autour d'un axe longitudinal de l'élément d'application (300) ;
e) appliquer le ciment osseux (500) à partir du récipient (500) par vissage de l'élément d'application (300) dans le récipient (500).

15. Procédé (800) selon la revendication 14, **caractérisé en ce que** le mouvement de rotation à l'étape d) déplace en rotation un premier cylindre creux (220) du système de commutation (200) contre un second cylindre creux (250) du système de commutation (200).

16. Procédé (800) selon la revendication 14 ou 15, **caractérisé en ce que** le premier cylindre creux (210) présente le premier filetage intérieur (220) et le second cylindre creux (250) présente un second filetage intérieur (260),
dans lequel le filetage extérieur (310) comprend au moins une rainure de filetage extérieur (315), le premier filetage intérieur (220) comprend au moins une première rainure de filetage intérieur (225) et le second filetage intérieur (260) comprend au moins une seconde rainure de filetage intérieur (265), et
l'au moins une rainure de filetage extérieur (315) divise le filetage extérieur (310) en sections de filetage extérieur (320),
l'au moins une première rainure de filetage intérieur (225) divise le premier filetage intérieur (220) en premières sections de filetage intérieur (230) et
l'au moins une seconde rainure de filetage intérieur (265) divise le second filetage intérieur (260) en secondes sections de filetage intérieur (270), respectivement le long de l'axe longitudinal (700), et
dans lequel l'au moins une première rainure de filetage intérieur (225) et l'au moins une seconde rainure de filetage intérieur (265) sont disposées, dans la seconde position de système de commutation, sur un axe sensiblement commun, de sorte qu'à l'étape c), les sections de filetage extérieur (320) sont déplacées dans le récipient (500) le long de l'axe commun.
